(19) Europäisches Patentamt / European Patent Office / Office européen des brevets

(11) **EP 1 415 667 B1**

(12) **EUROPEAN PATENT SPECIFICATION**

(45) Date of publication and mention of the grant of the patent:
**03.09.2008 Bulletin 2008/36**

(21) Application number: **02746091.4**

(22) Date of filing: **16.07.2002**

(51) Int Cl.:
***A61K 45/00*** *(2006.01)* ***A61K 31/66*** *(2006.01)*
***A61K 31/42*** *(2006.01)* ***A61P 1/18*** *(2006.01)*
***A61P 43/00*** *(2006.01)*

(86) International application number:
**PCT/JP2002/007213**

(87) International publication number:
**WO 2003/007991 (30.01.2003 Gazette 2003/05)**

(54) **PANCREATIC JUICE SECRETION REGULATORS COMPRISING LPA RECEPTOR CONTROLLER**

MITTEL ZUR REGULIERUNG DER SEKRETION VON PANKREASSAFT MIT EINEM LPA-REZEPTOR-REGLER

REGULATEURS DE LA SECRETION DE SUC PANCREATIQUE COMPRENANT UN AGENT DE REGULATION DU RECEPTEUR A LPA

(84) Designated Contracting States:
**AT BE BG CH CY CZ DE DK EE ES FI FR GB GR IE IT LI LU MC NL PT SE SK TR**

(30) Priority: **17.07.2001 JP 2001216133**

(43) Date of publication of application:
**06.05.2004 Bulletin 2004/19**


(73) Proprietor: **ONO PHARMACEUTICAL CO., LTD.**
**Osaka-shi, Osaka 541-8526 (JP)**

(72) Inventors:
- **NAKADE, Shinji,**
**Ono Pharmaceutical Co., Ltd.**
**-cho,**
**Mishima-gun,**
**Osaka 618-8585 (JP)**
- **SAGA, Hiroshi,**
**Ono Pharmaceutical Co., Ltd.**
**-cho,**
**Mishima-gun,**
**Osaka 618-8585 (JP)**

(74) Representative: **Henkel, Feiler & Hänzel**
**Patentanwälte**
**Maximiliansplatz 21**
**80333 München (DE)**



(56) References cited:
**EP-A- 1 364 659** **WO-A-99/35259**
**WO-A1-01/60819** **US-B1- 6 380 177**

- **BANDOH K. ET AL.: 'Molecular cloning and characterization of a novel human G-protein-coupled receptor, EDG7, for lysophosphatidic acid' J. BIOL. CHEM. vol. 274, no. 39, 1999, pages 27776 - 27785, XP000990175**
- **BANDOH K. ET AL.: 'Lysophosphatidic acid (LPA) receptors of the EDG family are differentially activated by LPA species. Structure-activity relationship of cloned LPA receptors' FEBS LETT. vol. 478, 2000, pages 1 - 2, 159-165, XP000990176**
- **IWATSUKI K. ET AL.: 'Effects of forskolin on pancreatic exocrine secretion and cyclic nucleotide concentrations of the dog pancreas' ARCH. INT. PHARMACODYN. THER. vol. 286, no. 2, 1987, pages 320 - 328, XP002957639**
- **SINGH J. ET AL.: 'Interaction between secretin and cholecystokinin-octapeptide in the exocrine rat pancreas in vivo and in vitro' EXP. PHYSIOL. vol. 77, no. 1, 1992, pages 191 - 204, XP002957640**
- **HEISE C.E. ET AL.: 'Activity of 2-substituted lysophosphatidic acid (LPA) analogs at LPA receptors: discovery of a LPA1/LPA3 receptor antagonist' MOL. PHARMACOL. vol. 60, no. 6, December 2001, pages 1173 - 1180, XP002957641**
- **AN S. ET AL.: 'Molecular cloning of the human Edg2 protein and its identification as a functional cellular receptor for lysophosphatidic acid' BIOCHEM. BIOPHYS. RES. COMMUN. vol. 231, no. 3, 1997, pages 619 - 622, XP002046899**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DUNLOP M. ET AL.: 'Association of cyclin-dependent kinase-4 and cyclin D1 in neonatal beta cells after mitogenic stimulation by lysophosphatidic acid' BIOCHEM. BIOPHYS. RES. COMMUN. vol. 218, no. 1, 1996, pages 132 - 136, XP002957642
- HOLLENBERG MORLEY D. (ATSUSHI KAWABATA) FOLIA PHARMACOL. JPN. vol. 114, no. 1, 1999, pages 173 - 179, XP002957643

## Description

Technical Field

The present invention relates to the use of a pharmaceutical composition for regulation of pancreatic juice secretion comprising a lysophosphatidic acid (hereinafter abbreviated to as LPA) receptor modulator. Particularly, it relates to the use of a pharmaceutical composition for inhibition of pancreatic juice secretion comprising an LPA receptor agonist, the use of a pharmaceutical composition for acceleration of pancreatic juice secretion comprising an LPA receptor antagonist, and the use of a pharmaceutical composition comprising them as the active ingredient. More particularly, it relates to the use of a pharmaceutical composition for inhibition of pancreatic juice secretion comprising an EDG-2 agonist, a pharmaceutical composition for acceleration of pancreatic juice secretion comprising an EDG-2 antagonist, and the use of a pharmaceutical composition comprising them as the active ingredient.

Background Art

It is known that various lipid mediators such as eicosanoid and platelet activating factor (PAF) are produced by the activity of phospholipase from cell membranes.

Lysophosphatidic acid, a lipid mediator, represented by formula (I)

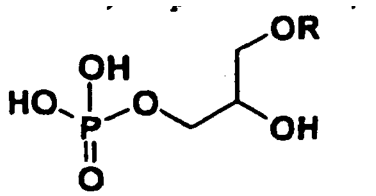

(I)

(wherein R is acyl, alkenyl or alkyl) is a lipid which is produced from cell membranes or in blood, acts as a mediator in the signal transduction system and delivers various signals into cells. LPA that exists naturally is L-α-LPA.

Recently, the existence of three subtypes of LPA receptor has been disclosed and it is gradually proved that their physiological activities are via LPA receptor. Three subtypes of LPA receptor are called EDG (Endothelial differentiation gene) -2, 4 and 7 ($LPA_1$, $LPA_2$ and $LPA_3$), respectively, and form part of EDG receptor family as well as EDG-1, 3, 5, 6 and 8 ($S1P_1$, $S1P_2$, $S1P_3$, $S1P_4$ and $S1P_6$) that are sphingosine-1-phosphate receptor. EDG-2 is called VZG-1, too (Mol Pharmacol, 2000 Dec; 58(6) : 1188-96). LPA receptor to which LPA binds delivers signals into cells via G-protein coupled receptor. As G proteins capable of binding to the LPA receptors, Gs, Gi, Gq, $G_{12/13}$ and the like are known, and the diversity is greatly involved in the action mechanism of LPA. In addition, EDGs-2, -4 and -7 which are widely distributed in organisms are different in a way of localization depending on their sub-types, and their role in receptors is accordingly considered diverse on tissues. However, the sub-type of receptors localized in various tissues has not yet been specified.

The known effects of LPA include *in vitro* fibroblast growth acceleration, smooth muscle contraction (J. Pharm. Pharmcol., 43, 774 (1991), J. Pharm. Pharmacol., 34, 514 (1982)), modulation of cytokine release from immunocytes, and the like. In addition, for the pharmacological effect caused by LPA *in vivo*, increase of blood pressure in rats and constriction of the airway in guinea pigs have been known.

On the other hand, it has been known about the effect of LPA on pancreas that LPA accelerates DNA synthesis on the beta cells of Langerhan's islet (BBRC, 218, 132 (1996)) and that LPA acts on ductal carcinoma cell lines to increase the level of intracellular calcium concentration (J. Cell Phys., 186, 53 (2001)). In addition, it has been known that the sub-type of LPA receptors, EDG-2 and EDG-7, are present in pancreas (Biochem. and Biophys. Res. Commun., 231, 619 (1997); J. Biol. Chem., 274, 39 2776 (1999)).

There is no report, however, as to the *in vitro* and *in vivo* activity of LPA on an acinal system and on the pancreatic juice secretion.

Heretofore, there is no report on a naturally occurring product inhibiting the pancreatic juice secretion but substances i.e., the sympathetic nervous transmitters of autonomic nervous system such as adrenalin, and hormones such as somatostatin, although physiologically active peptides such as cholecystokinin and secretin have been known as substances accelerating pancreatic juice secretion. Therefore, it was an unexpected finding that LPA which had not been classified into a neurotransmitter or hormone had an effect of inhibiting pancreatic juice secretion.

In general, it has been known that abnormally secreted pancreatic juice might cause a pathological feature of acute or chronic pancreatitis (Illustrative Digestive Organ Diseases, Series 14, Pancreatitis and Pancreatic Cancer, 90,

published by Medical View Co.). This has been considered that amylase and proteolytic enzymes contained in pancreatic juice could excessively act to destroy self-tissues. The known existing therapeutics for pancreatitis include camostat mesilate of which the effect is to inhibit proteolytic enzymes mainly including trypsin (Gendai Iryo, 16, 844 (1984)) and loxiglumide which inhibits the pancreatic juice secretion depending on cholecystokinin (Kan Tan Sui, 37(5), 703-728 (1998)).

On the other hand, among the enzymes contained in pancreatic juice, pancreatic lipase is an important lipolytic enzyme. In recent years, it has been elucidated that a pancreatic lipase inhibitor could inhibit absorption of fats and lipids from digestive organs, the effect being related with anti-obesity and resulting in launch of an anti-fat agent, orlistat, which inhibits pancreatic lipase (Expert Opinion Pharmacother, 1(4), 841-847, 2000 May,). Therefore, decrease of the pancreatic lipase level in pancreatic juice is expected to have the anti-obesity effect.

On the other hand, as ligands for LPA receptors or EDG-2, the following compounds have been known.

(1) US 6,380,177 discloses compounds represented by formula (A) are EDG-2 agonists:

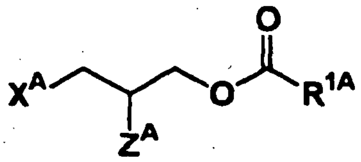 (A)

wherein $X^A$ represents a hydroxyl group,

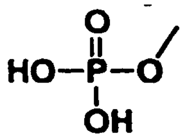 , 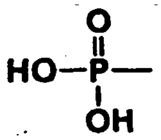 or 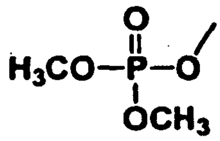

$Z^A$ represents hydrogen, bromine, chlorine, fluorine, iodine or $OR^{2A}$; $R^{2A}$ represents unsaturated alkyl group having 1 to 3 carbon atoms, and $R^{1A}$ represents saturated or unsaturated alkyl having 15 to 17 carbon atoms.

(2) Molecular Pharmacology, 60(6), 1173-1180 (2001) discloses compounds of formula (B) are agonists or antagonists for $LPA_1$ (EDG-2) and $LPA_3$ (EDG-7):

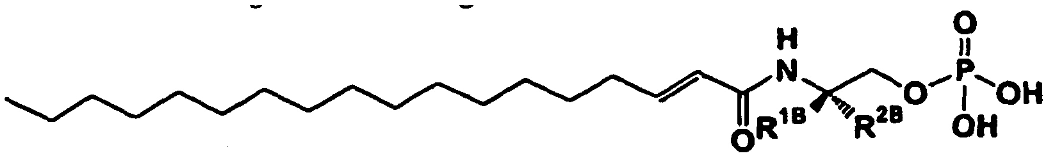 (B)

wherein one of $R^{1B}$ and $R^{2B}$ represents hydrogen, methylenehydroxy, carbomethyl, methylenamino, methyl, ethyl, isopropyl, benzyl or benzyl-4-oxybenzyl, and the other is necessarily hydrogen.

(3) WO 01/60819 discloses that compounds of formula (C) or salts thereof have an inhibitory effect to LPA receptors in an experiment using cells over-expressing EDG-2 and act on EDG-2:

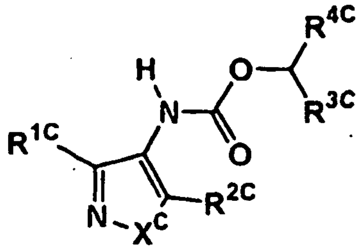 (C)

wherein $R^{1C}$ represents alkyl, aryl, a heterocyclic group, alkyloxy, aryloxy, alkylthio or arylthio which may have a substituent(s), or halogen;
$R^{2C}$ represents alkyl, aryl, a heterocyclic group, alkyloxy or aryloxy which may be have a substituent(s) or halogen;
$R^{3C}$ represents hydrogen atom, lower alkyl or halogenated alkyl;
$R^{4C}$ represents a group selected from the group consisting of (a) phenyl, aryl or a heterocyclic group which may have a substituent(s); (b) substituted or unsubstituted alkyl; and (c) substituted or unsubstituted alkenyl;
$X^C$ represents oxygen or sulfur, and
wherein $R^{3C}$ and $R^{4C}$ taken with the carbon atom to which they bond may form a 5- to 10-membered cyclic structure, and when $R^{3C}$ is a hydrogen atom, $R^{4C}$ is a group other than methyl.

Disclosure of the invention

The present inventors have studied on physiological activities of LPA receptor modulators in various ways in order to elucidate the role of LPA receptors and found unexpectedly that they act on pancreas and are involved in the pancreatic juice secretion. In addition, they have found that EDG-2 among the sub-type of LPA receptors is particularly involved in it. These were first ascertained by the present inventors in their experiment, though not expected from the prior art in the least.

Thus, the invention relates to the use of a pharmaceutical composition for regulation of pancreatic juice secretion in the following items (1) to (21) and a method for screening of modulators of LPA receptors (not claimed).

1. Use of a lysophosphatidic acid (LPA) receptor modulator for the preparation of a pharmaceutical composition for regulation of pancreatic juice secretion.

2. The use according to above-mentioned 1, wherein the LPA receptor modulator is an LPA receptor agonist.

3. The use according to above-mentioned 1 or 2, wherein the pharmaceutical composition has an activity of inhibiting pancreatic juice secretion.

4. Use of an LPA receptor agonist for the preparation of a pharmaceutical composition for treatment and/or prevention for pancreatic diseases or obesity.

5. The use according to above-mentioned 4, wherein the pancreatic disease is congenital exocrine dysfunction, acute pancretitis, chronic pancreatitis, pancreatic lithiasis, cholelithiasis, pancreatic tumor, pancreatic cyst or pancreatic diseases accompanied by abnormality in autonomic nervous system.

6. The use according to above-mentioned 2, wherein the LPA receptor agonist is 1-linolenoyl-lysophosphatidic acid or 1-oleoyl-lysophosphatidic acid.

7. The use According to above-mentioned 1, wherein the LPA receptor modulator is an LPA receptor antagonist.

8. The use according to above-meutioned 1 or 7, wherein the pharmaceutical composition has an activity of accelerating pancreatic juice secretion.

9. Use of an LPA receptor antagonist for the preparation of a pharmaceutical composition for treatment and/or prevention for digestive organ diseases.

10. The use according to above-mentioned 9, wherein the digestive organ disease is indigestion, constipation, diarrhea, cibophobia or malabsorption syndrome.

11. The use According to above-mentioned 2, wherein the LPA receptor is EDG-2.

12. The use according to above mentioned 11, wherein the EDG-2 agonist is a compound represented by formula (A):

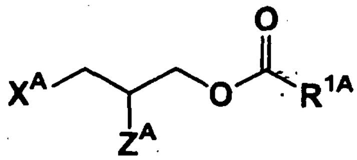 (A)

wherein $X^A$ represents hydroxyl,

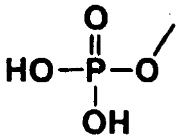 , 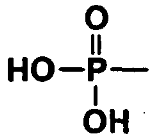 or 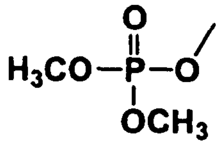 ,

$Z^A$ represents hydrogen, bromine, chlorine, fluorine, iodine or $OR^{2A}$;
$R^{2A}$ represents unsaturated alkyl having 1 to 3 carbon atoms; and
$R^{1A}$ represents saturated or unsaturated alkyl having 15 to 17 carbon atoms.

13. The use according to above-mentioned 11, wherein the EDG-2 agonist is a compound represented by formula (B):

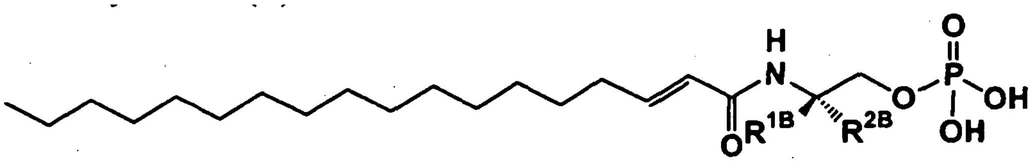 (B)

wherein one of $R^{1B}$ and $R^{2B}$ represents hydrogen, methylenehydroxy, carbomethyl, methylenamino, methyl, ethyl, isopropyl, benzyl, or benzyl-4-oxybenzyl, and the other is necessarily hydrogen.

14. The use according to above-mentioned 7, wherein the LPA receptor is EDG-2.

15. The use according to above- mentioned14, wherein the EDG-2 antagonist is a compound represented by formula (B):

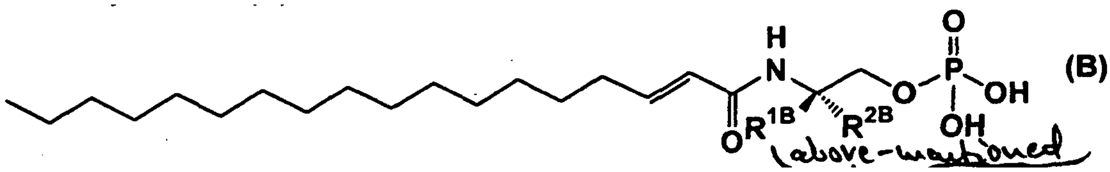

(B)

wherein all symbols have the same meanings as described in above-mentioned 13.

16. The use according to above-mentioned 14, wherein the EDG-2 antagonist is a compound represented by formula (C):

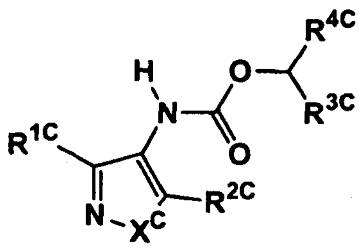 (C)

wherein $R^{1C}$ represents alkyl, aryl, a heterocyclic group, alkyloxy, aryloxy, alkylthio or arylthio which may have a substituent(s), or halogen;
$R^{2C}$ represents alkyl, aryl, a heterocyclic group, alkyloxy or aryloxy which may have a substituent(s), or halogen;
$R^{3C}$ represents hydrogen, lower alkyl or halogenated alkyl;
$R^{4C}$ represents a group selected from the group consisting of (a) phenyl, aryl or a heterocyclic group which may have a substituent(s); (b) substituted or unsubstituted alkyl; and (c) substituted or unsubstituted alkenyl;
$X^C$ represents oxygen or sulfur, and
wherein $R^{3C}$ and $R^{4C}$ taken with the carbon atom to which they bond may form a 5-to 10-membered cyclic structure, and when $R^{3C}$ is a hydrogen atom, $R^{4C}$ is a group other than methyl.

17. The use according to above-mentioned 16, wherein the EDG-2 antagonist is methyl 3-({4-[4-({[1-(2-chlorophenyl) ethoxy] carbonyl}amino)-3-methyl-5-isoxazolyl]benzyl}sulfanyl) propanoate.

18. Use of an LPA receptor agonist according to above-mentioned selected from the group consisting of 1-linolenoyl-lysophosphatidic acid, 1-oleoyl-lysophosphatidic acid, a compound represented by formula (A):

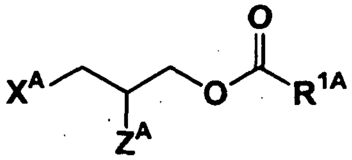

(A)

wherein $X^A$ represents hydroxyl,

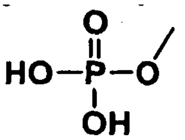

,

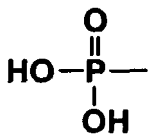

or

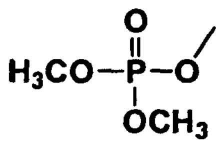

,

$Z^A$ represents hydrogen, bromine, chlorine, fluorine, iodine or $OR^{2A}$;
$R^{2A}$ represents unsaturated alkyl having 1 to 3 carbon atoms; and
$R^{1A}$ represents saturated or unsaturated alkyl having 15 to 17 carbon atoms, and a compound represented by formula (B):

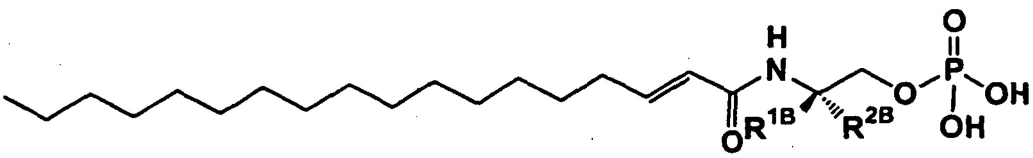

(B)

wherein one of $R^{1B}$ and $R^{2B}$ represents hydrogen, methylenehydroxy, carbomethyl, methylenamino, methyl, ethyl, isopropyl, benzyl, or benzyl-4-oxybenzyl, and the other is necessarily hydrogen,
for the preparation of a pharmaceutical composition for treatment and/or prevention for pancreatic diseases or obesity.

19. The use according to above-mentioned 18, wherein the pancreatic disease is congenital exocrine dysfunction, acute pancretitis, chronic pancreatitis, pancreatic lithiasis, cholelithiasis, pancreatic tumor, pancreatic cyst or pancreatic diseases accompanied by abnormality in autonomic nervous system.

20. Use of an LPA receptor antagonist according to above-mentioned 9 selected from the group consisting of a compound represented by the formula (B):

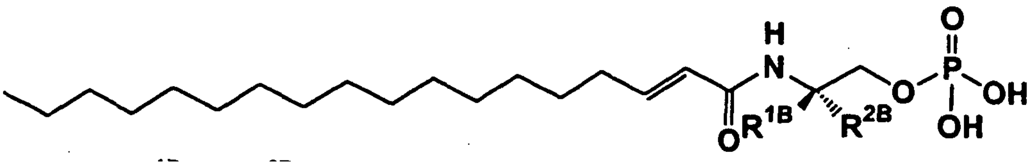
(B)

wherein one of $R^{1B}$ and $R^{2B}$ represents hydrogen, methylenehydroxy, carbomethyl, methylenamino, methyl, ethyl, isopropyl, benzyl or benzyl-4-oxybenzyl, and the other is necessarily hydrogen, and
a compound represented by formula (C):

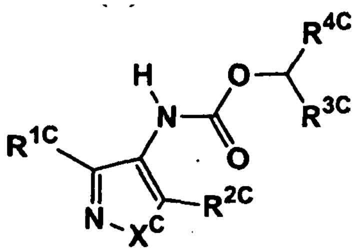
(C)

wherein $R^{1C}$ represents alkyl, aryl, a heterocyclic group, alkyloxy, aryloxy, alkylthio or arylthio which may have a substituent(s), or halogen;
$R^{2C}$ represents alkyl, aryl, a heterocyclic group, alkyloxy or aryloxy which may have a substituent(s), or halogen;
$R^{3C}$ represents hydrogen, lower alkyl or halogenated alkyl;
$R^{4C}$ represents a group selected from the group consisting of (a) phenyl, aryl or a heterocyclic group which may have a substituent(s); (b) substituted or unsubstituted alkyl; and (c) substituted or unsubstituted alkenyl;
$X^C$ represents oxygen or sulfur, and
wherein $R^{3C}$ and $R^{4C}$ taken with the carbon atom to which they bond may form a 5- to 10-membered cyclic structure, and when $R^{3C}$ is a hydrogen atom, $R^{4C}$ is a group other than methyl,
for the preparation of a pharmaceutical composition for treatment and/or prevention for digestive organ diseases.

21. The use according to above-mentioned 20, wherein the digestive organ disease is indigestion, constipation, diarrhea, cibophobia or malabsorption syndrome.

Detailed description

In the present invention, LPA means lysophosphatidic acid represented by formula (I) and it is a generic name of compounds in which one of two hydroxyl groups of glycerol in glycerophosphoric acid is substituted to fatty acid.
In this specification, the structural formula of LPA is represented by formula (I), and LPA also includes compounds in which the group R is bonded to the other hydroxyl group of glycerol, that is compounds of the following formula (I'):

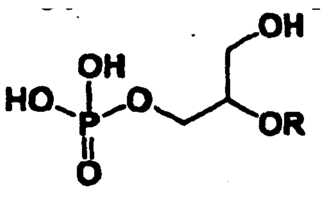
(I')

wherein R has the same meaning as described above.
In the present invention, LPA receptor modulator means LPA receptor agonist and LPA receptor antagonist.
The LPA receptor agonist includes all of naturally occurring and non-natural compounds which act on and activate the LPA receptors. The agonists are preferably low molecular compounds having a molecular weight of 100 to 700, more preferably LPA derivatives, more preferably 18:3-LPA (the compound of formula (I) wherein R is 1-linolenoyl) and 18:1-LPA (the compound of formula (I) wherein R is 1-oleoyl), and particularly 18:3-LPA. In addition, the following compounds are preferably used.

(1) Compounds of formula (A):

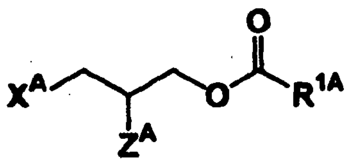 (A)

wherein all symbols have the same meanings as described above.

(2) Compounds of formula (B):

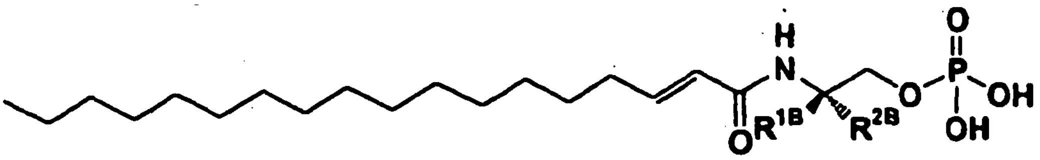 (B)

wherein all symbols have the same meanings as described above.

Among the LPAs represented by formula (I), a naturally occurring L-α-LPA is preferred.

Since the LPA receptor agonists have an effect inhibiting pancreatic juice secretion, they are useful in treatment and/or prevention of diseases caused by excessive pancreatic juice secretion, i.e., pancreatic diseases (congenital exocrine dysfunction, acute pancreatitis, chronic pancreatitis, pancreatic lithiasis, cholelithiasis, pancreatic tumor, pancreatic cyst) and obesity associated with pancreatic lipase.

Among the LPA receptor agonists, EDG-2 agonists are particularly preferred.

As the EDG-2 agonists, the compounds of formula (A) and of formula (B) are preferred. As for the EDG-2 agonists, the compounds which are expected to be found in future should be included naturally in addition to the so far known compounds.

Among the compounds represented by formula (A), preferred are those as described in US 6,380,177, specifically including LXR100023, LXR100008, LXR100016, LXR100024, LXR100001, LXR100013, and LXR100030.

Among the compounds represented by formula (B), preferred are those as described in Mol. Pharmacol., 60 (6), 1173-1180 (2001). Particularly preferred compounds are NAEPA, VPC31143, VPC31144, VPC31139, VPC31180, VPC12178, VPC12048, VPC12086, VPC12101, VPC12109, VPC12115, VPC12098, VPC40105, VPC12084, VPC12255, and VPC12204.

On the other hand, as LPA receptor antagonist, whatever inactivates LPA receptor is allowed.

Specifically, the above-mentioned compounds of formula (B) and of formula (C) are preferably used:

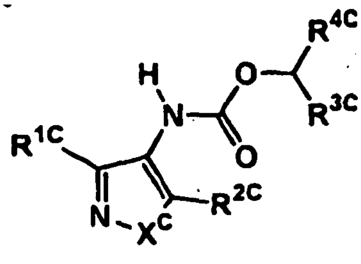 (C)

wherein all symbols have the same meanings as described above.

Since the LPA receptor antagonists have an effect promoting pancreatic juice secretion, they are useful in treatment and/or prevention of diseases caused by insufficient pancreatic juice secretion, i.e., indigestion, constipation, diarrhea, and cibophobia.

The EDG-2 antagonists do not accelerate the pancreatic juice secretion in normal animals (Example 6, Fig. 5). Thus, they are expected to have a good effect on decreased pancreatic juice secretion when digestive and absorptive function has been obstructed for a long term by surgery of digestive organs, hepato biliary disorder, pancreatic diseases or Crohn's disease, resulting in diarrhea and soft feces, poor absorption of various nutrients, and malnutrition (that is, malabsorption syndrome).

As the EDG-2 antagonists, the compounds represented by formulae (B) and (C) are preferred.

Among the compounds represented by formula (B), preferred are those as specifically described in Molecular Pharmacology, 60(6), 1173-1180 (2001). Particularly preferred compound is VPC12249.

Among the compounds represented by formula (C), preferred are those as described in Examples of WO01/60819. Particularly preferred is methyl 3-({4-[4-({[1-(2-chlorophenyl)ethoxy]earbonyl}amino)-3-methyl-5-isoxazolyl]benzyl}sulfanyl)propanoate.

The present inventors have confirmed that 18:3-LPA has a strong effect on pancreas *in vivo*. In particular, it has been found that administration of LPA inhibits pancreatic juice secretion (Example 1), reduces the volume of amylase and lipase secreted (Example 2 and Example 3), and moderates increase of pancreatic juice secretion induced by stimulation with cholecystokinin (CCK) (Example 4). CCK has been known as a factor to make pancreatitis worse or to give stress to a living body. Since LPA inhibits a CCK signal, it is considered that the effect of LPA might reduce the condition of pancreatitis caused by CCK and relieve from stress.

In this connection, no effect of LPA could not be confirmed in LPC which has the same carbon chain. This suggests that the effect of LPA is mediated through the LPA receptor.

In addition, it has been known that the subtype of LPA receptors include 3 kinds of EDGs (endothelial differentiation gene) 2, 4 and 7; thus, the compounds which act particularly on them are considered effective to pancreatic diseases.

Moreover, in an experiment using the EDG-2 agonist (Example 6), an inhibitory effect on pancreatic juice secretion was confirmed. In addition, in an experiment using the EDG-2 antagonist (Example 7), it was confirmed that the antagonist recovered the pancreatic juice secretion from the inhibition caused by 18:3-LPA and induced the secretion at the same level as in an untreated control group. Thus, this suggests that the effect is mediated through EDG-2.

Therefore, the LPA receptor antagonist, particularly EDG-2 antagonist, is expected to work to promote the pancreatic juice secretion. On the other hand, the LPA receptor agonist, particularly EDG-2 agonist, is expected to inhibit the pancreatic juice secretion.

The inhibitory effect of LPA on pancreatic juice secretion in pancreas found in the present invention suggests that LPA itself as well as the LPA receptor agonist and antagonist are useful as therapeutics in diseases caused by abnormal pancreatic juice secretion.

The method for determining the effect on the pancreatic juice secretion disclosed in the present invention is useful as an *in vivo* screening method for LPA receptor modulators, particularly EDG-2 modulators in mammals such as rats.

Toxicity:

The compound used in the present invention has low toxicity so that use of it as a pharmaceutical can be considered as safe enough.

Industrial Applicability

Application to pharmaceuticals:

The LPA receptor modulators used in the present invention, i.e., LPA receptor agonists and antagonists, bind to the LPA receptors to exhibit the effect of activation or inactivation. Therefore, they are considered useful in prevention and/or treatment of diseases caused by abnormal pancreatic juice secretion. Thus, the LPA receptor agonists, which inhibit pancreatic juice secretion, are considered useful in treatment and/or prevention of pancreatic diseases and obesity. The LPA receptor antagonists, which promote pancreatic juice secretion, are considered useful in treatment and/or prevention of indigestion, constipation, diarrhea and cibophobia.

The LPA receptor modulators used in the present invention are normally administered systemically or topically, and orally or parenterally for the above purpose.

In the present invention, LPA receptor modulators may be administered in combination with other drugs for the purpose of 1) complement and/or enhancement of preventing and/or treating effect, 2) improvement of dynamics and absorption of the compound, and lowering of dose, and/or 3) alleviation of side effect of the compound.

The LPA receptor modulators may be administered in combination with other drugs as a composition in one drug product comprising these components, or may be administered separately. When they are administered independently, they may be administered simultaneously or with time lag. Administering with time lag includes the method of administering LPA receptor modulators before other drugs and vice versa; they may be administered in the same route or not.

The above combination takes effects on whichever disease treating and/or preventing effect of LPA receptor modulators is complemented and/or enhanced.

As other drugs to complement and/or to enhance the preventing and/or treating effect of LPA receptor agonist

for pancreatic diseases (pancreatitis), for example, protease inhibitors, inhibitors of gastric-acid secretion, antispasmodic agents (COMT inhibitors, anti-serotonin agents *etc.*), nonsteroidal antiinflammatory drugs, centrally-acting analgesic agents, sedative drugs, digestive drugs, antacids, H2-blockers, anti-depressants, gastric mucosa anesthetic agents, function modulators of digestive tract (CCK-A antagonists), mitochondria benzodiazepine receptor antagonists *etc.* are given.

As other drugs to complement and/or to enhance the preventing and/or treating effect of LPA receptor agonist for obesity, for example, beta3 agonists, pancreatic lipase inhibitors *etc.* are given.

As beta3 agonists, SR-58611A, AJ-9677, KUL-7211, SB-418790, GW-427353, N-5984, SR-59062A *etc.* are given.

As pancreatic lipase inhibitors, orlistat *etc.* are given.

As other drugs to complement and/or to enhance the preventing and/or treating effect of LPA receptor antagonist for indigestion and/or indigestion syndrome, for example, antacids, H2-blockers, function modulators of digestive tract, function accelerators of digestive tract, antianxiety drug, tranquilizers, digestive drugs, proton pump inhibitors, antimuscarinic agents, anticholinergic drugs, defensive factor fortifiers, dopamine antagonists, digestive accelerators, drugs for controlling intestinal function, mitochondria benzodiazepine receptor antagonists *etc.* are given.

As other drugs to complement and/or to enhance the preventing and/or treating effect of LPA receptor antagonist for constipation, for example, evacuants *etc.* are given.

As evacuants, laxatives for small intestine (castor oil, olive oil), osmotic (saline) laxatives (magnesium sulfate, sodium sulfate, sodium hydrogenphosphate, artificial Karlsbad salt), bulk cathartics (methyl cellulose, carmellose sodium, agar), demulcent laxatives (liquid paraffin), infiltrative laxatives (dioctyl sodium sulfosuccinate), laxatives for large intestine (bisacodyl, phenovalin, sodium picosulfate, sennoside) *etc.* are given.

As other drugs to complement and/or to enhance the preventing and/or treating effect of LPA receptor antagonist for diarrhea, for example, antidiarrheal drugs *etc.* are given.

As antidiarrheal drugs, astringens (albumin tannate, bismuth subnitrate, bismuth subgallate), anticholinergic drugs (propantheline bromide, scopolia extract, scopolamine), opioidergic compounds (morphine, loperamide hydrochloride), absorbing agents (medicinal char, aluminum silicate) *etc.* are given.

As other drugs to complement and/or to enhance the preventing and/or treating effect of LPA receptor antagonist for cibophobia, for example, SSRI (selective serotonin re-uptake inhibitor), SNRI (selective serotonin and norepinephrine re-uptake inhibitor) *etc.* are given.

As SSRI, fluoxetine hydrochloride, fluvoxamine maleate *etc.* are given.

As other SSRI, minaprine hydrochloride, sibutramine hydrochloride, tramadole hydrochloride, venlafaxine hydrochloride, paroxetine hydrochloride, milnacipran hydrochloride, citalopram hydrobromide, nefazolon hydrochloride, celtraline hydrochloride, esitalopram, duloxetine hydrochloride, tramadol hydrochloride *etc.* are given.

As SNRI, venlafexine *etc.* are given.

Weight ratio of LPA receptor modulator and other drugs is not limited. Other drugs may be administered as a combination of any two or more drugs.

In other drugs to complement and/or to enhance the preventing and/or treating effect of LPA receptor modulator, drugs that not only exist now but also may be found in the future on the basis of above mechanisms are included.

LPA receptor modulator with other drugs are used for the above-described purpose, it is usually administered systemically or topically via an oral or parenteral route.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, or from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The LPA receptor modulator or LPA receptor modulator and other drugs may be administered in the composition of, for example, solid compositions, liquid compositions or other compositions each for oral administration, or injections, liniments or suppositories, each for parenteral administration.

Solid compositions for oral administration include compressed tablets, pills, capsules, powders and granules. Capsules include hard capsules and soft capsules.

In such solid compositions, one or more of the active substance(s) may be used as it stands or as pharmaceuticals by the law of the art in combination with diluting agent (lactose, mannitol, glucose, microcrystallite cellulose, starch *etc.*), binder (hydroxypropylcellulose, polyvinylpyrrolidone, magnesium aluminometasilicate *etc.*), disintegrants (cellulose calcium glycolate *etc.*), lubricants (magnesium stearate *etc.*), stabilizer and solubilizing agent (glutamic acid, aspartic acid *etc.*) *etc.*. And it may be coated with a coating agents (sucrose, gelatin, hydroxypropyl cellulose, hydroxypropyl methylcellulose phthalate *etc.*), or with two or more layers, if necessary. Furthermore, capsules made of a substance

which can be absorbed in the body, for example, gelatin, are included.

Liquid compositions for oral administration include pharmaceutically acceptable solutions, suspensions, emulsions, syrups and elixirs. In such liquid compositions, one or more of the active substance(s) may be solved, suspended or emulsified in generally used inert diluent(s) (purified water, ethanol or mixtures thereof *etc.*). The compositions may comprise, in addition to the inert diluent, humectants, suspending agents, emulsifying agent, sweetening agents, flavoring agents, aromatic agents preservatives and buffer agents.

In the parenteral administration, formulation of external use include, for example, ointment, ger, cream, poultice, patch, liniment, atomized agent, inhalation, spray, eye drops and nasal drops *etc.* They includes one or more of the active compound(s) and be prepared by known method or usual method.

Ointment is prepared by known method or usual method. For example, it is prepared by levigation or fusion of one or more of the active compound(s) and substrate. The substrate of ointment is selected from known or usual one. For example, higher fatty acid or higher fatty acid ester (adipic acid, myristic acid, palmitic acid, stearic acid, oleic acid, adipic acid ester, myristic acid ester, palmitic acid ester, stearic acid ester, oleic acid ester *etc.*), wax (yellow beeswax, Spermaceti, ceresin *etc.*), surfactant (polyoxyethylene alkyl ether phosphoric acid ester *etc.*), higher alcohol (cetanol, stearil alcohol, cetostearyl alcohol *etc.*), silicon oil (dimethyl polysiloxane *etc.*), hydrocarbon (hydrophilic petrolatum, white petrolatum, purified lanolin, liquid paraffin *etc.*), glycol (ethylene glycol, diethylene glycol, propylene glycol, polyethylene glycol, macrogol *etc.*), vegetable oil (castor oil, olive oil, sesame oil, turpentine oil *etc.*), animal oil (mink oil, egg yolk oil, squalane, squalene *etc.*), water, absorption accelerator, skin fit inhibitor, *etc.* are used as single substance selected from them or mixture which consists of two or more kinds that is selected from them. Moreover, humectant, preservative agent, stabilizer, antioxidative agent, fragrant materials *etc.* may be contained.

Gel is prepared by known method or usual method. For example, it is prepared by fusion of one or more of the active compound(s) and substrate. The substrate of gel is selected from known or usual one. For example, lower alcohol (ethanol, isopropylalcohol, *etc.*), gelling agent (carboxy methyl cellulose, hydroxy ethyl cellulose, hydroxy propyl cellulose, ethyl cellulose, *etc.*), neutralizing agent (triethanolamine, diisopropanolamine, *etc.*), surfactant (monostearate, polyethylene glycol, *etc.*), gum, water, absorption accelerator, skin fit inhibitor, *etc.* are used as single substance selected from them or mixture which consists of two or more kinds that is selected from them. Moreover, preservative agent, antioxidative agent, fragrant materials, *etc.* may be contained.

Cream is prepared by known method or usual method. For example, it is prepared by fusion or emulsification of one or more of the active compound(s) and substrate. The substrate of cream is selected from known or usual one. For example, higher fatty acid ester, lower alcohol, hydrocarbon, polyalcohol (propylene glycol, 1,3-butylene glycol, *etc.*), higher alcohol (2-hexyldecanol, cetanol, *etc.*), emulsifying agent (polyoxyethylene alkyl ether, fatty acid ester, *etc.*), water, absorption accelerator, skin fit inhibitor, *etc.* are used as single substance selected from them or mixture which consists of two or more kinds that is selected from them. Moreover, preservative agent, antioxidative agent, fragrant materials, *etc.* may be contained.

Poultice is prepared by known method or usual method. For example, it is prepared by fusion of one or more of the active compound(s) and substrate, and then the kneaded one is laid over support medium. The substrate for poultice is selected from known or usual one. For example, thickening agent (polyacrylic acid, polyvinylpyrolidone, gum arabia, starch, gelatin, methyl cellulose, *etc.*), humectant (urea, glycerin, propylene glycol, *etc.*), bulking agent (kaolin, zinc oxide, talc, calcium, magnesium, *etc.*), water, solubilizing agent, thickener, skin fit inhibitor, *etc.* are used as single substance selected from them or mixture which consists of two or more kinds that is selected from them. Moreover, preservative agent, antioxidative agent, fragrant materials, *etc.* may be contained.

Patch is prepared by known method or usual method. For example, it is prepared by fusion of one or more of the active compound(s) and substrate, and then laid over support medium. The substrate for patch is selected from known or usual one. For example, polymer substrate, fat, higher fatty acid, thickener, skin fit inhibitor, *etc.* are used as single substance selected from them or mixture which consists of two or more kinds that is selected from them. Moreover, preservative agent, antioxidative agent, fragrant materials, *etc.* may be contained.

Liniment is prepared by known method or usual method. For example, one or more of the active compound(s) may be dissolved, suspended or emulsified in water, alcohol (ethanol, polyethylene glycol, *etc.*), higher fatty acid, glycerin, soap, emulsifying agent, suspending agent, *etc.* as single substance selected from them or mixture which consists of two or more kinds that is selected from them. Moreover, preservative agent, antioxidative agent, fragrant materials, *etc.* may be contained.

Atomized agent, inhalation and spray may comprise in addition to a generally used diluent, a stabilizer such as sodium bisulfite and an isotonization buffer such as sodium chloride, sodium citrate or citric acid. The preparation process of sprays is described in detail in, for example, U.S. Patent No. 2,868,691 and 3,095,355.

Injections for parenteral administration include aqueous, suspensions, emulsions and solid forms which are dissolved or suspended into solvent(s) for injection immediately before use. In injections, one or more of the active compound(s) may be dissolved, suspended or emulsified into solvent(s). The solvents may include distilled water for injection, physiological salt solution, vegetable oil, propylene glycol, polyethylene glycol, alcohol such as ethanol, or a

mixture thereof Moreover, these injections may comprise some additives, such as stabilizing agents, solution adjuvants (such as glutamic acid, aspartic acid or POLYSORBATE80 (registered trade mark), *etc.*), suspending agents, emulsifying agents, soothing agent, buffering agents, preservative. They may be sterilized at a final step, or may be prepared and compensated according to sterile methods. They may also be manufactured in the form of sterile solid forms, for example, freeze-dried products, which may be dissolved in sterile water or some other sterile diluent(s) for injection immediately before use.

The dosage form of eye drops for parenteral administration include ophthalmic solutions, ophthalmic suspensions, ophthalmic emulsions, ophthalmic solutions dissolved before use and eye ointments.

Such eye drops are prepared in a known method. For example, one or more of the active compound(s) may be dissolved, suspended or emulsified in solvent(s). As solvent for eye drops, for example, distilled water for injection, physiological salt solution, other aqueous solvent, non-aqueous solvent for injection (for example, vegetable oil *etc.*) and combination thereof are given. If necessary, eye drops may comprise one or more additives such as isotonizing agent (such as sodium chloride or concentrated glycerin), a buffering agent (such as sodium phosphate or sodium acetate), a surfactant (such as "Polysorbate 80" (trade name), polyoxyl 40 stearate or polyoxyethylene hydrogenated castor oil), a stabilizer (such as sodium citrate or edetate sodium) and an antiseptic (such as benzalkonium chloride or p-aminobenzonic acid). They may be sterilized at a final step, or may be prepared and compensated according to sterile methods. They may also be manufactured in the form of sterile solid forms, for example, freeze-dried products, which may be dissolved in sterile water or some other sterile diluent(s) immediately before use.

The dosage of inhalations for parenteral administration include aerosol, powders for inhalation or liquids for inhalation. The liquids for inhalation may be dissolved or suspended in water or the other appropriate solvent as needed.

Such inhalations are prepared in a known method. For example, a liquid for inhalation is prepared by using proper additives selected from an antiseptic (such as benzalkonium chloride or p-aminobenzonic acid), a coloring agent, a buffering agent (such as sodium phosphate or sodium acetate), an isotonizing agent (such as sodium chloride or concentrated glycerin), thickening agent (such as carboxyvinylpolymer), or an accelerator of absorption, *etc.*, if necessary.

A powder for inhalation is prepared by using proper additives selected from a lubricant agent (such as stearin acid and the salt thereof), a binding agent, (such as starch, dextrin), a diluting agent (such as lactose, cellulose), a coloring agent, an antiseptic (such as benzalkonium chloride or p-aminobenzonic acid), an accelerator of absorption, *etc.*, if necessary.

In case of administration of liquid for inhalation, spray (atomizer, nebulizer) is usually used and in case of administration of powder for inhalation, inhalation administration apparatus for powder agents is usually used.

The other compositions for parenteral administration include suppositories for intrarectal administration and pessaries for vaginal administration which comprise one or more of the active substance(s) and may be prepared by methods known per se.

Brief description of the drawings

Figure.1 is a graph which shows an inhibitory effect of LPA on pancreatic juice secretion of rat.

Figure.2 is a graph which shows an inhibitory effect of LPA on amylase secretion of rat.

Figure.3 is a graph which shows an inhibitory effect of LPA on lipase secretion of rat.

Figure.4 is a graph which shows an inhibitory effect of LPA on cholecystokinin-induced pancreatic juice secretion of rat.

Figure.5 is a graph which shows an recovery effect of methyl 3-({4-[4-({[1-(2-chlorophenyl)ethoxy]carbonyl}amino)-3-methyl-5-isoxazolyl]benzyl}sulfanyl)propanoate against 18:3 LPA-induced inhibition of pancreatic juice secretion of rat.

Best mode for carrying out the invention

The present invention will be described by reference example and example.

Reference example 1 : The preparation of 1-linolenoyl (18:3)-LPA

A composition which contains 1-linolenoyl (18:3)-LPC (lysophosphatidyl choline) (SRL-B641) 3mg/mL, phospholipase D (Sigma P-8023) 60 U/mL, 200mM Tris-HCl pH7.5, and 5mM sodium fluoride was reacted enzymatically overnight with stirring strongly at 37°C. It was extracted with mixed solvent of chloroform and methanol (once in the proportion of chloroform : methanol = 2 : 1, and then twice in the proportion of chloroform : methanol = 17 : 3), and pH was adjusted to 2.5 with the addition of methanol and 1N hydrochloric acid accordingly in upper layer. It was extracted

twice with mixed solvent of chloroform : methanol = 17 : 3, and chloroform layer was collected and concentrated. The residue was neutralized with chloroform-methanol-3% ammonia water (6 : 5 : 1) and concentrated to give 1-linolenoyl (18:3)-LPA.

Furthermore, by the same procedure, Lysophosphatidic acid (LPA), if desired, can be prepared using a corresponding lysophosphatidyl choline (LPC).

Example 1 : Determination of the amount of pancreatic juice secreted in rats

Male rats (8 to 9 week-old) fasting from the morning of the previous day were anesthetized by subcutaneous application of urethane (1.2 g/5ml/kg) and polyethylene tubes were inserted into the arteria carotis communis and the femoral vein. The abdomen was cut open along the median line and the gastric pylorus was ligated. Then, a polyethylene tube was inserted into the bile duct at the hepatic portal portion, through which the tip of the tube was inserted into the pore opened in the duodenum intestine in order to return bile to the duodenum intestine. The origin of the hepatoapancreatic tube from the duodenum intestine was cut open, into which a tube was inserted in the same way as in the bile duct and led outside the body. Pure pancreatic juice was collected from the tube.

The drug was continuously administered through the femoral vein with saline. On the other hand, a saline containing 18:3-LPA, 18:3-LPC or 0.5% bovine serum albumin (Sigma, A-0281) as control (vehicle) was infused (1 ml/h) through the cervical vein, and pancreatic juice was collected for 1 hour. Dose-dependent inhibitory effect of LPA on the pancreatic juice secretion in rats was confirmed. The test was conducted according to the protocol as shown in Table 1.

Table 1

| *i.v.* infusion: 1ml/h | | | Pre-Admn.(1hr) | | Admn. (1hr) | |
|---|---|---|---|---|---|---|
| Gp | (n) | Major Drug Adm n. | cervical vein | femoral vein | cervical vein | femoral vein |
| A | 4 | *Vehicle* | saline | saline | *vehicle* | saline |
| B | 4 | 18:3-LPA (1mg/kg/h) | saline | saline | LPA | saline |
| C | 5 | 18:3-LPA (3mg/kg/h) | saline | saline | LPA | saline |
| D | 4 | 18:3-LPA (10mg/kg/h) | saline | saline | LPA | saline |
| E | 5 | 18:3-LPC (10mg/kg/h) | saline | saline | LPC | saline |

The volume of the collected pancreatic juice was measured. In this connection, saline was infused through the cervical vein and the femoral vein 1 hour before administration of the drug, during which time the amount of pancreatic juice secreted was measured in order to ascertain that there was no considerable difference between individual animals in the pre-value to the pancreatic juice secretion in the individual animals.

The pancreatic juice secretion in rats was decreased by 18:3-LPA, of which the effect on LPA varied dose-dependently and reached approximately the maximum by i.v. infusion at 3mg/kg/h and 1ml/h (Fig. 1). On the other hand, the effect of another phospholipid LPC having the same fatty acid side chain was examined and it was found that 18:3-LPC (10mg/kg/h, 1ml/h, i.v. infusion) had no inhibitory effect on the pancreatic juice secretion as observed in LPA. Thus, it is considered that LPA works to inhibit the pancreatic juice secretion through the LPA receptors.

Example 2 : Determination of the amount of amylase secreted

The pancreatic juice collected in Example 1 was diluted with Dulbecco's phosphate-buffered saline(-) (PBS(-)) at an appropriate dilution rate, of which the amylase activity was determined with a reagent of a Diacolor AMY Neorate (Ono Pharmaceutical) kit. To 10μl of an amylase standard solution (Worthington Biochemical, diluted to 100U/L to 6,400U/L with PBS(-)) or the collected diluted pancreatic juice was added 50μl of an enzyme reagent of the kit, and the mixture was incubated at 37°C for 3 minutes. Then, after addition of 50μl of a substrate reagent of the kit, the variation (Vmax) of optical density (OD) at 400nm was monitored at 37°C for 3 minutes (SPECTRAMAX 250). The amylase activity was calculated from an amylase standard curve to evaluate the effect of the subject substance by means of the secreted amount of the enzyme (U/h) obtained by the enzyme activity (concentration) (U/L) multiplied by the amount of pancreatic juice collected (L/h). Fig. 2 shows the results.

The amount of amylase secreted was reduced by administration of LPA at 1, 3 or 10mg/kg/h. On the other hand, the effect of a phospholipid LPC which has the same fatty acid side chain was examined, indicating that 18:3-LPC (10mg/kg/h, 1ml/h, i.v. infusion) had no inhibitory effect on the amylase secretion in rats as observed in LPA. Thus, it is considered that LPA works to inhibit the amylase secretion through the LPA receptors as described above.

Example 3 : Determination of the amount of lipase secreted

The pancreatic juice collected in Example 1 was diluted with saline at an appropriate dilution rate and the lipase activity was measured with a reagent contained in Liquitech Lipase Color kit (Roche Diagnostics). Then, 100μl of a kit R-1 solution was added to 10μl of a lipase standard solution (Worthington Biochemical; prepared to 1U/L to 1,000U/L with saline) or the collected diluted pancreatic juice, and the mixture was incubated at 37°C for 5 minutes. Then, 60μl of a kit R-2 solution was added and the variation (Vmax) of optical density (OD) at 570-700nm was monitored at 37°C for 3 minutes (SPECTRAMAX 250). The lipase activity of the pancreatic juice was calculated from a lipase standard curve to evaluate the effect of the subject substance by means of the secreted amount of the enzyme (U/h) obtained by the enzyme activity (concentration)(U/L) multiplied by the amount of pancreatic juice collected (L/h). Fig. 3 shows the results.

LPA reduced significantly the amount of lipase secreted at 3 and 10mg/kg/h. On the other hand, the effect of a phospholipid LPC which has the same fatty acid side chain was examined, indicating that 18:3-LPC (10mg/kg/h, 1ml/h, i.v. infusion) had no inhibitory effect on the lipase secretion in rats as observed in LPA. Thus, it is considered that LPA works to inhibit the lipase secretion through the LPA receptors.

Example 4 : Determination of the amount of rat's pancreatic juice secreted by stimulation with cholecytokinin (CCK)

The pancreatic juice secreted in rats was collected in the same manner as described in Example 1. In order to ascertain the effect of LPA on excessive pancreatic juice secretion by CCK, an experiment was conducted according to a protocol as shown in Table 2. As CCK, CCK-8 (Sigma No.C-2175) was used.

Table 2

| *i.v.* infusion: 1ml/h | | | Pre-Admn.(1hr) | | Admn.1 (1hr) | | Admn.2 (1hr) | |
|---|---|---|---|---|---|---|---|---|
| Gp | (n) | Mainly administered drug | cervical vein | femoral vein | cervical vein | femoral vein | cervical vein | femoral vein |
| A | 5 | No stimuli Control | saline | saline | saline | saline | 0.5%BSA /saline | saline |
| B | 4 | *vehicle* | saline | saline | saline | saline | CCK-8 | saline |
| C | 4 | 18:3-LPA (3mg/kg/h) | saline | saline | saline | LPA | CCK-8 | LPA |
| D | 4 | 18:3-LPC (10mg/kg/h) | saline | saline | saline | LPC | CCK-8 | LPC |

Saline was infused through the cervical vein and the femoral vein 1 hour before administration of the drug, during which time the amount of pancreatic juice secreted was measured in order to ascertain that there was no considerable difference between individual animals in the pre-value to the pancreatic juice secretion in the individual animals. After 1 hour infusion of saline, to the 18:3-LPA group and the 18:3-LPC group, were infused 3mg/kg/h of 18:3-LPA and 10mg/kg/h of 18:3-LPC, respectively, through the femoral vein. After lapse of 1 hour, CCK-8 was intravenously infused for stimulation through the cervical vein in order to examine the effect of LPA on the excessive pancreatic juice secretion. To the control group (vehicle) with no stimuli by CCK-8, 0.5% BSA in saline was administered through the cervical vein. On the other hand, saline was infused to the LPA control group (vehicle) through the femoral vein. In other groups, CCK-8 was administered through the cervical vein at a rate of 0.12μg/kg/h and 1ml/h. In the 18:3-LPA group and the 18:3-LPC group, 3mg/kg/h of 18:3-LPA and 10mg/kg/h of 18:3-LPC, respectively, were infused through the femoral vein. In the control group with no stimuli by CCK-8 and in the 18:3-LPA control group (vehicle), saline was administered through the femoral vein. One hour after the final administration (Admn.2), the volume of pancreatic juice collected was measured. Fig. 4 shows the results.

Secretion of pancreatic juice in rats was markedly increased by CCK-8. 18:3-LPA (3mg/kg/h) in intravenous infusion reduced the increased pancreatic juice secretion approximately to the control level. In this connection, the effect of another phospholipid lysophosphatidylcholine (LPC) having the same fatty acid side chain was examined and it was found that 18:3-LPC (10mg/kg/h, 1ml/h, intravenous infusion) had no inhibitory effect on the pancreatic juice secretion induced by CCK-8 as observed in 18:3-LPA. Thus, it is considered that the effect of LPA is mediated through the LPA receptors.

Example 5 : Evaluation of the antagonistic and agonistic activities to EDG-2

The effect on EDG-2 was proved, for example, by the following experiment.

Using Chinese hamster ovary (CHO) cells which expressed excessively human EDG-2 gene, the activity of said receptor antagonist was evaluated. The cells expressing EDG-2 were cultured using a Ham's F12 medium (GIBCO BRL) containing 10% FBS (fetal bovine serum), penicillin/streptomycin, and blasticidin (5 $\mu$g/ml). In order to incorporate Fura2-AM (Dojindo) into the cells, the cells were incubated in a 5$\mu$M Fura2-AM solution [Ham's F12 medium containing 10% FBS, HEPES buffer (20 mM, pH7.4), and probenecid (2.5 mM, Sigma, No.P-8761)] at 37°C for 60 minutes, and then washed once with a Hank's solution containing HEPES buffer (20mM, pH7.4) and probenecid (2.5mM) and immersed into the Hank's solution. Subsequently, evaluation was made according to the following method (i) or (ii).

(i) Evaluation of the antagonist activity

A plate was set on a fluorescent drug screening system (Hamamatsu Photonics), and after measurement for 30 seconds with no stimulation, a solution of a compound to be tested was added. After lapse of 5 minutes, 18:3-LPA (final concentration: 100nM) was added, the intracellular calcium ion concentration before and after the addition was measured every 3 seconds (excitation wave length: 340nm and 380nm; fluorescent wave length: 500nm). The compound was dissolved in DMSO and added so as to be 1nM to 10$\mu$M of the final concentration. 18:3-LPA (1-linolenoyl LPA) was synthesized from 18:3-LPC (1-linolenoyl lisophosphatidylcholine) (Sedary) using phospholipase D. The antagonizing activity of EDG-2 was calculated as an inhibition rate (%) by the following equation, wherein the peak value of LPA (final concentration: 100nM) in a well into which DMSO containing no Compound 1 was added was regarded as a control value (A), and in the cells treated with the compound the difference (B) between the value before addition of the compound and that after the addition was obtained and compared with the control value.

$$\text{Inhibition rate (\%)} = ((A-B) / A) \times 100$$

The $IC_{50}$ value was calculated as a concentration of the compound of the present invention which showed 50% inhibition.

A compound as described in WO01/60819, that is, methyl 3-({4-[4-({[1-(2-chlorophenyl)ethoxy]carbonyl}amino)-3-methyl-5-isoxazolyl]benzyl}sulfanyl)propanoate (referred to as Compound 1) was employed as a subject compound, and the inhibitory activity to EDG-2 was determined. $IC_{50}$ of Compound 1 was 1.5 $\mu$M.

(ii) Determination of the agonist activity

A plate was set on the above-mentioned fluorescent drug screening system, and after measurement for 30 seconds with no stimulation, a solution of a compound to be tested was added. The compound to be evaluated was dissolved in DMSO and added so as to be 0.1 nM to 10 $\mu$M of the final concentration, i.e., 1/1000 concentration as a DMSO solution. Then, the intracellular $Ca^{2+}$ concentration (Fura2-$Ca^{2+}$ fluorescence) was measured every 3 seconds (excitation wave length: 340nm and 380nm; fluorescent wave length: 500nm).

In measurement of the agonist activity, the peak value obtained by stimulation with 18:3-LPA (1-linolenoyl LPA) in a well into which DMSO was added instead of the compound to be assessed was used as a control value (A); the latter was compared with an increased value (B) derived from the fluorescent ratio between the fluorescence before addition of the subject compound and that after the addition. Thus, the increased rate of the intracellular $Ca^{2+}$ concentration was calculated from:

$$\text{Increase rate (\%)} = (B/A) \times 100$$

$EC_{50}$ value was calculated from the increased rate at each level of the concentrations of the compound.

Example 6 : Effect of the LPA receptor or EDG-2 agonist on the pancreatic juice secretion in rats

The effect of EDG-2 agonist on the pancreatic juice secretion in rats can be confirmed, for example, according to the following method.

Using an LPA receptor or EDG-2 agonist, the effect of LPA on the pancreatic juice secretion was examined.

An LPA receptor agonist 18:3-LPA was intravenously infused to a rat through the cervical vein, and the amount of pancreatic juice secreted was measured. In a group to which 18:3-LPA was administered, the pancreatic juice secretion was inhibited in rats.

In the same manner, it was confirmed that the compound having an EDG-2 agonist activity inhibited the pancreatic juice secretion.

Example 7 : Effect of the LPA receptor or EDG-2 antagonist on the pancreatic juice secretion in rats

The effect of the LPA receptor or EDG-2 antagonist on the pancreatic juice secretion can be confirmed according to the following method.

Using Compound 1 having the effect as a EDC-2 antagonist was confirmed in Example 5, the effect of LPA on the pancreatic juice secretion was examined.

In rats, (1) 18:3-LPA and (2) saline as a control (vehicle) for 18:3-LPA were respectively infused through the femoral vein, and (3) Compound 1 and (4) 5% bovine serum albumin/10% DMSO/saline as a control (vehicle) for Compound 1 were respectively infused through the cervical vein, at a rate of 0.5 ml/h for 1 hour.

On the other hand, saline was previously infused through the femoral vein and cervical vein (0.5 ml/h), and after one hour the pancreatic juice was collected in order to ascertain that there was no difference in the initial volumes of the secreted pancreatic juice in rats in each group.

Fig.5 shows the results. In a group to which Compound 1 (10mg/kg/h, i.v. infusion) alone was administered, there was no difference in the basal (at resting) secretion volume of pancreatic juice from that of the control group (vehicle) (5% bovine serum albumin/10% DMSO/saline) after one hour. In a group to which 18:3-LPA (3 mg/kg/h, i.v. infusion) was administered, inhibition of the basal secretion volume was observed as described above. For this inhibition, when Compound 1 was administered together with 18:3-LPA, the pancreatic juice secretion was significantly recovered from the inhibition by 18:3-LPA. This strongly suggests that the inhibition of pancreatic juice secretion by 18:3-LPA is achieved through EDG-2.

Preparation Example 1

The following components were admixed in a conventional method, punched out to give 100 tablets each containing 50 mg of active ingredient.

| | |
|---|---|
| · 18:3-LPA (1-linolenoyl lysophosphatidic acid) | 5.0g |
| · calcium carboxymethylcellulose (disintegrant) | 0.2g |
| · magnesium stearate (lubricant) | 0.1g |
| · microcrystalline cellulose | 4.7g |

Preparation Example 2

After mixing the following components by a conventional method, the resulting solution was sterilized by a conventional method and 5 ml portions thereof were filled in amples, respectively, and freeze-dried by a conventional method to obtain 100 amples for injection containing each 20 mg of the active ingredient.

| | |
|---|---|
| · 18 : 3-LPA | 2.0 mg |
| · Mannitol | 20 g |
| · Distilled water | 1000 ml |

Preparation Example 3

The following components were admixed in a conventional method, punched out to give 100 tablets each containing 50 mg of active ingredient.

| | |
|---|---|
| · methyl 3-({4-[4-({[1-(2-chlorophenyl)ethoxy]carbonyl}amino)-3-methyl-5-isoxazolyl]benzyl}sulfanyl) propanoate | 5.0g |
| · calcium carboxymethylcellulose (disintegrant) | 0.2g |
| · magnesium stearate (lubricant) | 0.1g |
| · microcrystalline cellulose | 4.7g |

Preparation Example 4

After mixing the following components by a conventional method, the resulting solution was sterilized by a conventional method and 5 ml portions thereof were filled in amples, respectively, and freeze-dried by a conventional method to obtain 100 amples for injection containing each 20 mg of the active ingredient.

| | |
|---|---|
| · methyl 3-({4-[4-({[1-(2-chlorophenyl)ethoxy]carbonyl}amino)-3-methyl-5-isoxazolyl]benzyl}sulfanyl) propanoate | 2.0 g |
| · Mannitol | 20 g |
| · Distilled water | 1000 ml |

## Claims

1. Use of a lysophosphatidic acid (LPA) receptor modulator for the preparation of a pharmaceutical composition for regulation of pancreatic juice secretion.

2. The use according to above- mentioned 1, wherein the LPA receptor modulator is an LPA receptor agonist.

3. The use according to above- mentioned 1 or 2, wherein the pharmaceutical composition has an activity of inhibiting pancreatic juice secretion.

4. Use of an LPA receptor agonist for the preparation of a pharmaceutical composition for treatment and/or prevention for pancreatic diseases or obesity.

5. The use according to above- mentioned 4, wherein the pancreatic disease is congenital exocrine dysfunction, acute pancretitis, chronic pancreatitis, pancreatic lithiasis, cholelithiasis, pancreatic tumor, pancreatic cyst or pancreatic diseases accompanied by abnormality in autonomic nervous system.

6. The use according to above- mentioned 2, wherein the LPA receptor agonist is 1-linolenoyl-lysophosphatidic acid or 1-oleoyl-lysophosphatidic acid.

7. The use according to above- mentioned 1, wherein the LPA receptor modulator is an LPA receptor antagonist.

8. The use according to above- mentioned 1 or 7, wherein the pharmaceutical composition has an activity of accelerating pancreatic juice secretion.

9. Use of an LPA receptor antagonist for the preparation of a pharmaceutical composition for treatment and/or prevention for digestive organ diseases.

10. The use according to above- mentioned 9, wherein the digestive organ disease is indigestion, constipation, diarrhea, cibophobia or malabsorption syndrome.

11. The use according to above- mentioned 2, wherein the LPA receptor is EDG-2.

12. The use according to above- mentioned 11, wherein the EDG-2 agonist is a compound represented by formula (A):

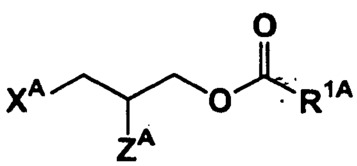

(A) ;

wherein $X^A$ represents hydroxyl,

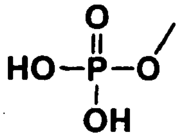 , 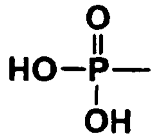 or 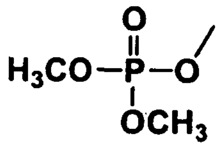 ,

$Z^A$ represents hydrogen, bromine, chlorine, fluorine, iodine or $OR^{2A}$;
$R^{2A}$ represents unsaturated alkyl having 1 to 3 carbon atoms; and
$R^{1A}$ represents saturated or unsaturated alkyl having 15 to 17 carbon atoms.

**13.** The use according to above- mentioned 11, wherein the EDG-2 agonist is a compound represented by formula (B):

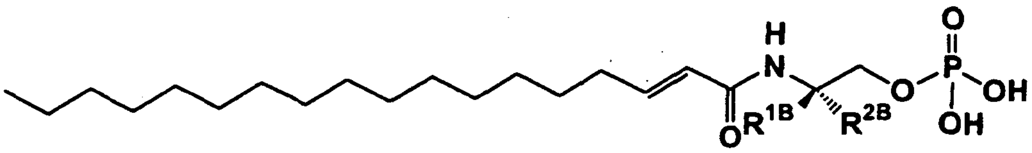 (B)

wherein one of $R^{1B}$ and $R^{2B}$ represents hydrogen, methylenehydroxy, carbomethyl, methylenamino, methyl, ethyl, isopropyl, benzyl or benzyl-4-oxybenzyl, and the other is necessarily hydrogen.

**14.** The use according to above- mentioned 7, wherein the LPA receptor is EDG-2.

**15.** The use according to above- mentioned 14, wherein the EDG-2 antagonist is a compound represented by formula (B):

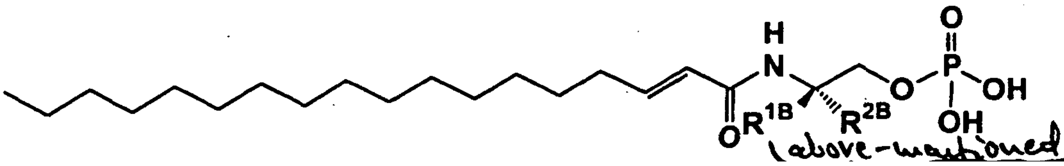 (B)

(above-mentioned)

wherein all symbols have the same meanings as described in above-meutioned 13.

**16.** The use according to above- mentioned 14, wherein the EDG-2 antagonist is a compound represented by formula (C):

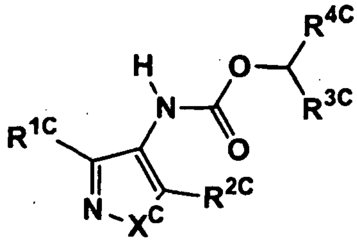 (C)

wherein $R^{1C}$ represents alkyl, aryl, a heterocyclic group, alkyloxy, aryloxy, alkylthio or arylthio which may have a substituent(s), or halogen;
$R^{2C}$ represents alkyl, aryl, a heterocyclic group, alkyloxy or aryloxy which may have a substituent(s), or halogen;
$R^{3C}$ represents hydrogen, lower alkyl or halogenated alkyl;
$R^{4C}$ represents a group selected from the group consisting of (a) phenyl, aryl or a heterocyclic group which may have a substituent(s); (b) substituted or unsubstituted alkyl; and (c) substituted or unsubstituted alkenyl;
$X^C$ represents oxygen or sulfur, and
wherein $R^{3C}$ and $R^{4C}$ taken with the carbon atom to which they bond may form a 5-to 10-membered cyclic structure, and when $R^{3C}$ is a hydrogen atom, $R^{4C}$ is a group other than methyl.

**17.** The use according to above- mentioned 16, wherein the EDG-2 antagonist is methyl 3-({4-[4({[1-(2-chlorophenyl)

ethoxy] carbonyl}amino)-3-methyl-5-isoxazolyl]benzyl}sulfanyl) propanoate.

**18.** Use of an LPA receptor agonist according to above- mentioned 4 selected from the group consisting of 1-linolenoyl-lysophosphatidic acid, 1-oleoyl-lysophosphatidic acid, a compound represented by formula (A):

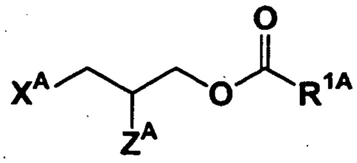 (A)

wherein $X^A$ represents hydroxyl,

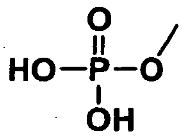 , 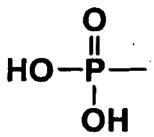 or 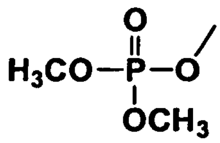 ,

$Z^A$ represents hydrogen, bromine, chlorine, fluorine, iodine or $OR^{2A}$;
$R^{2A}$ represents unsaturated alkyl having 1 to 3 carbon atoms; and
$R^{1A}$ represents saturated or unsaturated alkyl having 15 to 17 carbon atoms, and a compound represented by formula (B):

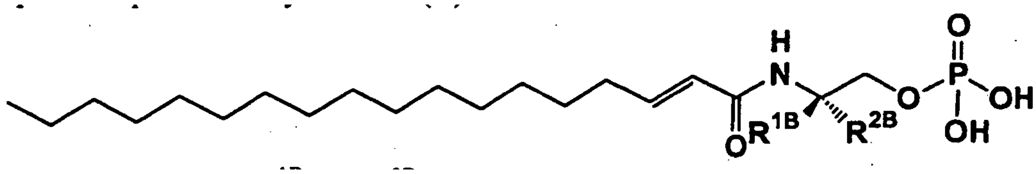 (B)

wherein one of $R^{1B}$ and $R^{2B}$ represents hydrogen, methylenehydroxy, carbomethyl, methylenamino, methyl, ethyl, isopropyl, benzyl, or benzyl-4-oxybenzyl, and the other is necessarily hydrogen,
for the preparation of a pharmaceutical composition for treatment and/or prevention for pancreatic diseases or obesity.

**19.** The use according to above-mentioned 18, wherein the pancreatic disease is congenital exocrine dysfunction, acute pancretitis, chronic pancreatitis, pancreatic lithiasis, cholelithiasis, pancreatic tumor, pancreatic cyst or pancreatic diseases accompanied by abnormality in autonomic nervous system.

**20.** Use of an LPA receptor antagonist according to above- mentioned selected from the group consisting of a compound represented by the formula (B):

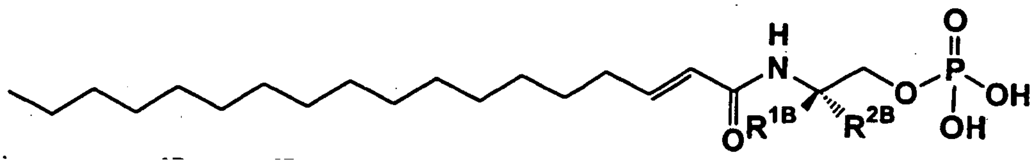 (B)

wherein one of $R^{1B}$ and $R^{2B}$ represents hydrogen, methylenehydroxy, carbomethyl, methylenamino, methyl, ethyl, isopropyl, benzyl or benzyl-4-oxybenzyl, and the other is necessarily hydrogen, and
a compound represented by formula (C):

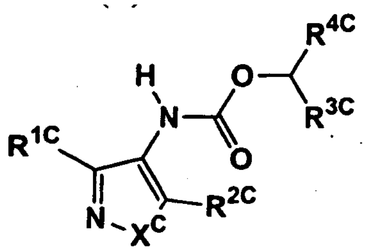

(C)

wherein $R^{1C}$ represents alkyl, aryl, a heterocyclic group, alkyloxy, aryloxy, alkylthio or arylthio which may have a substituent(s), or halogen;
$R^{2C}$ represents alkyl, aryl, a heterocyclic group, alkyloxy or aryloxy which may have a substituent(s), or halogen;
$R^{3C}$ represents hydrogen, lower alkyl or halogenated alkyl;
$R^{4C}$ represents a group selected from the group consisting of (a) phenyl, aryl or a heterocyclic group which may have a substituent(s); (b) substituted or unsubstituted alkyl; and (c) substituted or unsubstituted alkenyl;
$X^C$ represents oxygen or sulfur, and
wherein $R^{3C}$ and $R^{4C}$ taken with the carbon atom to which they bond may form a 5- to 10-membered cyclic structure, and when $R^{3C}$ is a hydrogen atom, $R^{4C}$ is a group other than methyl,
for the preparation of a pharmaceutical composition for treatment and/or prevention for digestive organ disease.

**21.** The use according to above-mentioned 20, wherein the digestive organ disease is indigestion, constipation, diarrhea, cibophobia or malabsorption syndrome.

**Patentansprüche**

**1.** Verwendung eines Lysophosphatidsäure(LPA)rezeptormodu-lators zur Herstellung einer pharmazeutischen Zusammensetzung zur Regulation der Pankreassaftsekretion.

**2.** Verwendung nach Anspruch 1, wobei der LPA-Rezeptormodulator ein LPA-Rezeptoragonist ist.

**3.** Verwendung nach Anspruch 1 oder 2, wobei die pharmazeutische Zusammensetzung die Aktivität einer Hemmung der Pankreassaftsekretion aufweist.

**4.** Verwendung eines LPA-Rezeptoragonisten zur Herstellung einer pharmazeutischen Zusammensetzung zur Behandlung und/oder Prävention von Pankreaserkrankungen oder Fettsucht.

**5.** Verwendung nach Anspruch 4, wobei die Pankreaserkrankung eine angeborene exokrine Dysfunktion, akute Pankreatitis, chronische Pankreatitis, Pankreaslithiasis, Cholelithiasis, ein Pankreaskarzinom, eine Pankreaszyste oder Pankreaserkrankungen, die von einer Anomalität des autonomen Nervensystems begleitet sind, ist.

**6.** Verwendung nach Anspruch 2, wobei der LPA-Rezeptoragonist 1-Linolenoyl-lysophosphatidsäure oder 1-Oleoyllysophosphatidsäure ist.

**7.** Verwendung nach Anspruch 1, wobei der LPA-Rezeptormodulator ein LPA-Rezeptorantagonist ist.

**8.** Verwendung nach Anspruch 1 oder 7, wobei die pharmazeutische Zusammensetzung die Aktivität einer Beschleunigung der Pankreassaftsekretion aufweist.

**9.** Verwendung eines LPA-Rezeptorantagonisten zur Herstellung einer pharmazeutischen Zusammensetzung zur Behandlung und/oder Prävention von Verdauungsorganerkrankungen.

**10.** Verwendung nach Anspruch 9, wobei die Verdauungsorganerkrankung Indigestion, Obstipation, Diarrhoe, Cibophobie oder Malabsorptionssyndrom ist.

**11.** Verwendung nach Anspruch 2, wobei der LPA-Rezeptor EDG-2 ist.

**12.** Verwendung nach Anspruch 11, wobei der EDG-2-Agonist eine Verbindung der Formel (A) ist:

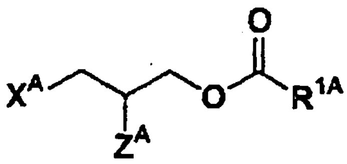 (A)

worin $X^A$ für Hydroxyl,

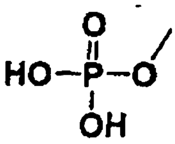 , 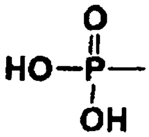 oder 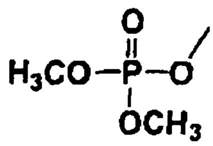 ,

steht,
$Z^A$ für Wasserstoff, Brom, Chlor, Fluor, Iod oder $OR^{2A}$ steht,
$R^{2A}$ für ungesättigtes Alkyl mit 1 bis 3 Kohlenstoffatomen steht, und
$R^{1A}$ für gesättigtes oder ungesättigtes Alkyl mit 15 bis 17 Kohlenstoffatomen steht.

**13.** Verwendung nach Anspruch 11, wobei der EDG-2-Agonist für eine Verbindung der Formel (B) steht:

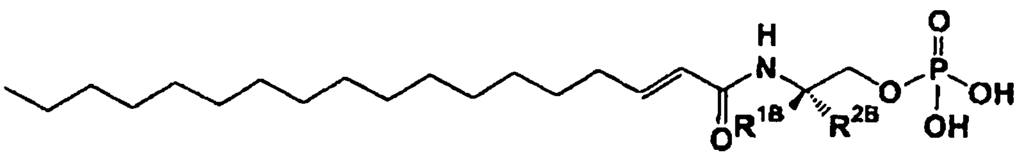 (B)

worin ein Rest von $R^{1B}$ und $R^{2B}$ für Wasserstoff, Methylenhydroxy, Carbomethyl, Methylenamino, Methyl, Ethyl, Isopropyl, Benzyl oder Benzyl-4-oxybenzyl steht und der andere zwangsläufig für Wasserstoff steht.

**14.** Verwendung nach Anspruch 7, wobei der LPA-Rezeptor EDG-2 ist.

**15.** Verwendung nach Anspruch 14, wobei der EDG-2-Antagonist eine Verbindung der Formel (B) ist:

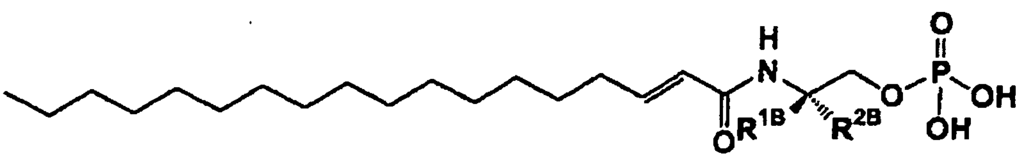 (B)

worin alle Symbole die in Anspruch 13 beschriebenen Bedeutungen aufweisen.

**16.** Verwendung nach Anspruch 14, wobei der EDG-2-Antagonist eine Verbindung der Formel (C) ist:

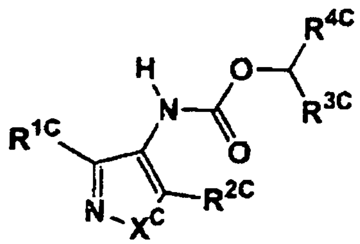 (C)

worin $R^{1C}$ für Alkyl, Aryl, eine heterocyclische Gruppe, Alkyloxy, Aryloxy, Alkylthio oder Arylthio, die (einen) Substituenten aufweisen können, oder Halogen steht;
$R^{2C}$ für Alkyl, Aryl, eine heterocyclische Gruppe, Alkyloxy oder Aryloxy, die (einen) Substituenten aufweisen können, oder Halogen steht;
$R^{3C}$ für Wasserstoff, Niederalkyl oder halogeniertes Alkyl steht;
$R^{4C}$ für eine Gruppe steht, die aus der Gruppe von (a) Phenyl, Aryl oder einer heterocyclischen Gruppe, die (einen) Substituenten aufweisen können; (b) substituiertem oder unsubstituiertem Alkyl und (c) substituiertem oder unsubstituiertem Alkenyl ausgewählt ist;
$X^C$ für Sauerstoff oder Schwefel steht, und
worin $R^{3C}$ und $R^{4C}$ zusammengenommen mit dem Kohlenstoffatom, an das sie gebunden sind, eine 5- bis 10-gliedrige cyclische Struktur bilden können, und, wenn $R^{3C}$ für ein Wasserstoffatom steht, $R^{4C}$ für eine andere Gruppe als Methyl steht.

**17.** Verwendung nach Anspruch 16, wobei der EDG-2-Antagonist Methyl-3-({4-[4({[1-(2-chlorphenyl)ethoxy]carbonyl}-amino)-3-methyl-5-isoxazolyl]benzyl}sulfanyl)propanoat ist.

**18.** Verwendung eines LPA-Rezeptoragonisten nach Anspruch 4, der aus der Gruppe von 1-Linolenoyl-lysophosphatidsäure, 1-Oleoyl-lysophosphatidsäure, einer Verbindung der Formel (A):

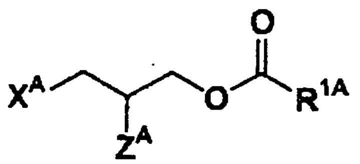 (A)

worin $X^A$ für Hydroxyl,

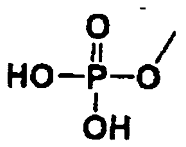 , 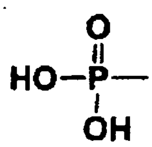 oder 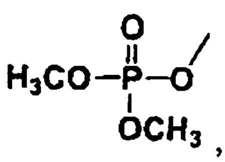 ,

steht,
$Z^A$ für Wasserstoff, Brom, Chlor, Fluor, Iod oder $OR^{2A}$ steht,
$R^{2A}$ für ungesättigtes Alkyl mit 1 bis 3 Kohlenstoffatomen steht, und
$R^{1A}$ für gesättigtes oder ungesättigtes Alkyl mit 15 bis 17 Kohlenstoffatomen steht,
und einer Verbindung der Formel (B):

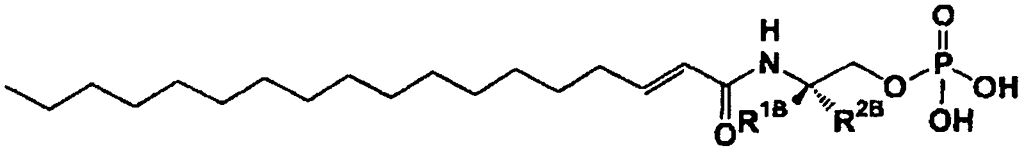

(B)

worin ein Rest von $R^{1B}$ und $R^{2B}$ für Wasserstoff, Methylenhydroxy, Carbomethyl, Methylenamino, Methyl, Ethyl, Isopropyl, Benzyl oder Benzyl-4-oxybenzyl steht und der andere zwangsläufig für Wasserstoff steht, ausgewählt ist, zur Herstellung einer pharmazeutischen Zusammensetzung zur Behandlung und/oder Prävention von Pankreaserkrankungen oder Fettsucht.

**19.** Verwendung nach Anspruch 18, wobei die Pankreaserkrankung eine angeborene exokrine Dysfunktion, akute Pankreatitis, chronische Pankreatitis, Pankreaslithiasis, Cholelithiasis, ein Pankreaskarzinom, eine Pankreaszyste oder Pankreaserkrankungen, die von einer Anomalität des autonomen Nervensystems begleitet sind, ist.

**20.** Verwendung eines LPA-Rezeptorantagonisten nach Anspruch 9, der aus der Gruppe von einer Verbindung der Formel (B) :

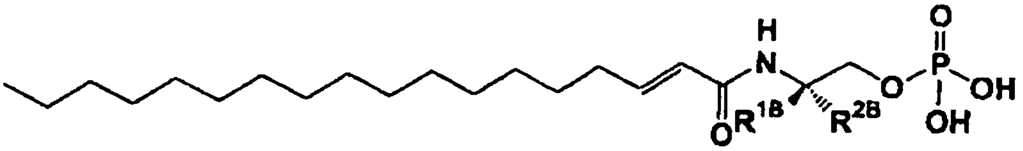

(B)

worin ein Rest von $R^{1B}$ und $R^{2B}$ für Wasserstoff, Methylenhydroxy, Carbomethyl, Methylenamino, Methyl, Ethyl, Isopropyl, Benzyl oder Benzyl-4-oxybenzyl steht und der andere zwangsläufig für Wasserstoff steht, und einer Verbindung der Formel (C):

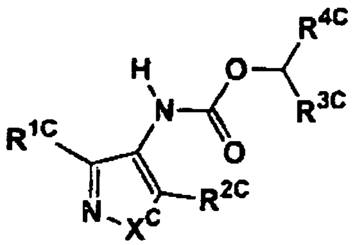

(C)

worin $R^{1C}$ für Alkyl, Aryl, eine heterocyclische Gruppe, Alkyloxy, Aryloxy, Alkylthio oder Arylthio, die (einen) Substituenten aufweisen können, oder Halogen steht;
$R^{2C}$ für Alkyl, Aryl, eine heterocyclische Gruppe, Alkyloxy oder Aryloxy, die (einen) Substituenten aufweisen können, oder Halogen steht;
$R^{3C}$ für Wasserstoff, Niederalkyl oder halogeniertes Alkyl steht;
$R^{4C}$ für eine Gruppe steht, die aus der Gruppe von (a) Phenyl, Aryl oder einer heterocyclischen Gruppe, die (einen) Substituenten aufweisen können; (b) substituiertem oder unsubstituiertem Alkyl und (c) substituiertem oder unsubstituiertem Alkenyl ausgewählt ist;
$X^C$ für Sauerstoff oder Schwefel steht, und
worin $R^{3C}$ und $R^{4C}$ zusammengenommen mit dem Kohlenstoffatom, an das sie gebunden sind, eine 5- bis 10-gliedrige cyclische Struktur bilden können, und, wenn $R^{3C}$ für ein Wasserstoffatom steht, $R^{4C}$ für eine andere Gruppe als Methyl steht,
ausgewählt ist, zur Herstellung einer pharmazeutischen Zusammensetzung zur Behandlung und/oder Prävention von Verdauungsorganerkrankungen.

21. Verwendung nach Anspruch 20, wobei die Verdauungsorganerkrankung Indigestion, Obstipation, Diarrhoe, Cibophobie oder Malabsorptionssyndrom ist.

## Revendications

1. Utilisation d'un modulateur du récepteur de l'acide lysophosphatidique (LPA) pour la préparation d'une composition pharmaceutique destinée à réguler la sécrétion de suc pancréatique.

2. Utilisation selon la revendication 1, dans laquelle le modulateur du récepteur du LPA est un agoniste du récepteur du LPA.

3. Utilisation selon la revendication 1 ou 2, dans laquelle la composition pharmaceutique a une activité d'inhibition de la sécrétion de suc pancréatique.

4. Utilisation d'un agoniste du récepteur du LPA pour la préparation d'une composition pharmaceutique destinée au traitement et/ou à la prévention de maladies pancréatiques ou de l'obésité.

5. Utilisation selon la revendication 4, dans laquelle la maladie pancréatique est la dysfonction exocrine congénitale, la pancréatite aiguë, la pancréatite chronique, la lithiase pancréatique, la cholélithiase, une tumeur pancréatique, un kyste pancréatique ou des maladies pancréatiques accompagnées d'une anomalie du système nerveux autonome.

6. Utilisation selon la revendication 2, dans laquelle l'agoniste du récepteur du LPA est l'acide 1-linolénoyl-lysophosphatidique ou l'acide 1-oléoyl-lysophosphatidique.

7. Utilisation selon la revendication 1, dans laquelle le modulateur du récepteur du LPA est un antagoniste du récepteur du LPA.

8. Utilisation selon la revendication 1 ou 7, dans laquelle la composition pharmaceutique a une activité d'accélération de la sécrétion de suc pancréatique.

9. Utilisation d'un antagoniste du récepteur du LPA pour la préparation d'une composition pharmaceutique destinée au traitement et/ou à la prévention de maladies des organes du système digestif.

10. Utilisation selon la revendication 9, dans laquelle la maladie des organes du système digestif est une indigestion, la constipation, la diarrhée, la cibophobie ou le syndrome de malabsorption.

11. Utilisation selon la revendication 2, dans laquelle le récepteur du LPA est EDG-2.

12. Utilisation selon la revendication 11, dans laquelle l'agoniste d'EDG-2 est un composé représenté par la formule (A) :

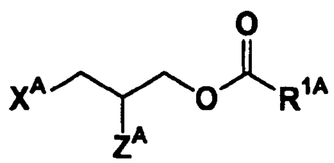

(A)

dans laquelle $X^A$ représente un groupe hydroxyle,

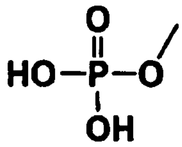 , 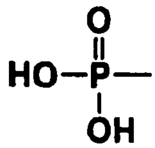 ou 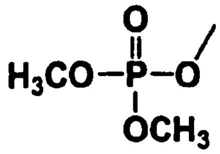 ,

$Z^A$ représente un atome d'hydrogène, de brome, de chlore, de fluor, d'iode ou $OR^{2A}$ ;
$R^{2A}$ représente un groupe alkyle insaturé ayant de 1 à 3 atomes de carbone ; et
$R^{1A}$ représente un groupe alkyle saturé ou insaturé ayant de 15 à 17 atomes de carbone.

**13.** Utilisation selon la revendication 11, dans laquelle l'agoniste d'EDG-2 est un composé représenté par la formule (B) :

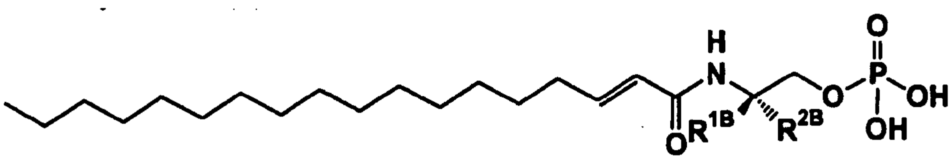 (B)

dans laquelle l'un de $R^{1B}$ et $R^{2B}$ représente un atome d'hydrogène, un groupe méthylènehydroxy, carbométhyle, méthylènamino, méthyle, éthyle, isopropyle, benzyle ou benzyl-4-oxybenzyle, et l'autre est nécessairement un atome d'hydrogène.

**14.** Utilisation selon la revendication 7, dans laquelle le récepteur du LPA est EDG-2.

**15.** Utilisation selon la revendication 14, dans laquelle l'antagoniste d'EDG-2 est un composé représenté par la formule (B) :

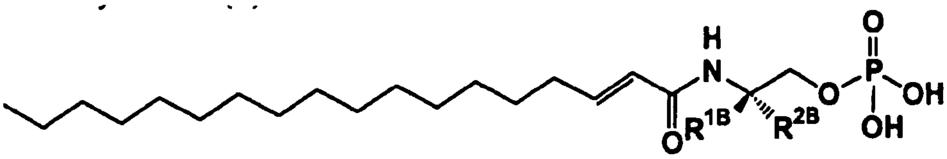 (B)

dans laquelle tous les symboles ont les mêmes significations que celles décrites dans la revendication 13.

**16.** Utilisation selon la revendication 14, dans laquelle l'antagoniste d'EDG-2 est un composé représenté par la formule (C) :

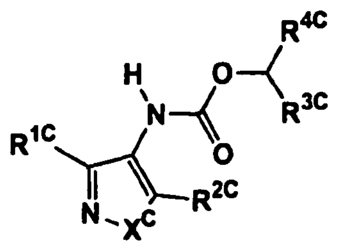 (C)

dans laquelle $R^{1C}$ représente un groupe alkyle, aryle, un groupe hétérocyclique, alkyloxy, aryloxy, alkylthio ou arylthio qui peut avoir un (des) substituant(s), ou un atome d'halogène ;
$R^{2C}$ représente un groupe alkyle, aryle, un groupe hétérocyclique, alkyloxy ou aryloxy qui peut avoir un (des)

substituant(s), ou un atome d'halogène ;
$R^{3C}$ représente un atome d'hydrogène, un groupe alkyle inférieur ou alkyle halogéné ;
$R^{4C}$ représente un groupe choisi dans le groupe constitué par (a) un groupe phényle, aryle ou un groupe hétérocyclique, qui peut avoir un (des) substituant(s) ; (b) un groupe alkyle substitué ou non substitué ; et (c) un groupe alcényle substitué ou non substitué ;
$X^C$ représente un atome d'oxygène ou de soufre, et
dans laquelle $R^{3C}$ et $R^{4C}$ pris avec l'atome de carbone auquel ils sont liés peuvent former une structure cyclique à 5 à 10 chaînons, et dans laquelle $R^{3C}$ est un atome d'hydrogène, $R^{4C}$ est un groupe autre qu'un groupe méthyle.

**17.** Utilisation selon la revendication 16, dans laquelle l'antagoniste d'EDG-2 est le 3-({4-[4-({[1-(2-chlorophényl)éthoxy]carbonyl}amino)-3-méthyl-5-isoxazolyl]benzyl}sulfanyl)propanoate de méthyle.

**18.** Utilisation d'un agoniste du récepteur du LPA selon la revendication 4, choisi dans le groupe constitué par l'acide 1-linolénoyl-lysophosphatidique, l'acide 1-oléoyl-lysophosphatidique, un composé représenté par la formule (A) :

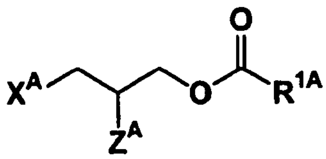 (A)

dans laquelle $X^A$ représente un groupe hydroxyle,

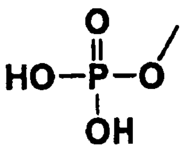 , 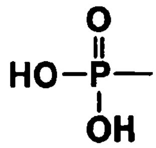 ou 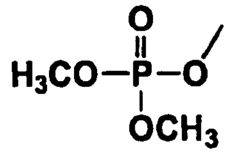 ,

$Z^A$ représente un atome d'hydrogène, de brome, de chlore, de fluor, d'iode ou $OR^{2A}$ ;
$R^{2A}$ représente un groupe alkyle insaturé ayant de 1 à 3 atomes de carbone ; et
$R^{1A}$ représente un groupe alkyle saturé ou insaturé ayant de 15 à 17 atomes de carbone, et un composé représenté par la formule (B) :

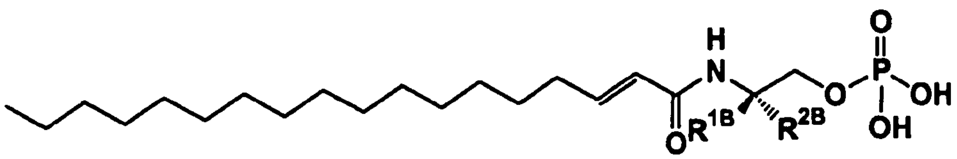 (B)

dans laquelle l'un de $R^{1B}$ et $R^{2B}$ représente un atome d'hydrogène, un groupe méthylènehydroxy, carbométhyle, méthylènamino, méthyle, éthyle, isopropyle, benzyle ou benzyl-4-oxybenzyle, et l'autre est nécessairement un atome d'hydrogène,
pour la préparation d'une composition pharmaceutique destinée au traitement et/ou à la prévention d'une maladie pancréatique ou de l'obésité.

**19.** Utilisation selon la revendication 18, dans laquelle la maladie pancréatique est la dysfonction exocrine congénitale, la pancréatite aiguë, la pancréatite chronique, la lithiase pancréatique, la cholélithiase, une tumeur pancréatique, un kyste pancréatique ou des maladies pancréatiques accompagnées d'une anomalie du système nerveux autonome.

**20.** Utilisation d'un antagoniste du récepteur du LPA selon la revendication 9, choisi dans le groupe constitué par un composé représenté par la formule (B) :

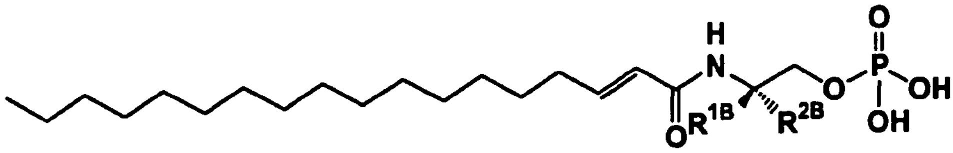

(B)

dans laquelle l'un de $R^{1B}$ et $R^{2B}$ représente un atome d'hydrogène, un groupe méthylènehydroxy, carbométhyle, méthylènamino, méthyle, éthyle, isopropyle, benzyle ou benzyl-4-oxybenzyle, et l'autre est nécessairement un atome d'hydrogène, et
un composé représenté par la formule (C) :

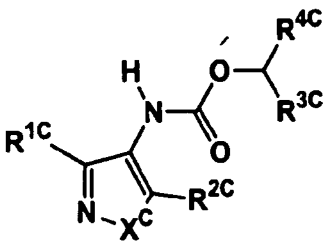

(C)

dans laquelle $R^{1C}$ représente un groupe alkyle, aryle, un groupe hétérocyclique, alkyloxy, aryloxy, alkylthio ou arylthio qui peut avoir un (des) substituant(s), ou un atome d'halogène ;
$R^{2C}$ représente un groupe alkyle, aryle, un groupe hétérocyclique, alkyloxy ou aryloxy qui peut avoir un (des) substituant(s), ou un atome d'halogène ;
$R^{3C}$ représente un atome d'hydrogène, un groupe alkyle inférieur ou alkyle halogéné ;
$R^{4C}$ représente un groupe choisi dans le groupe constitué par (a) un groupe phényle, aryle ou un groupe hétérocyclique, qui peut avoir un (des) substituant(s) ; (b) un groupe alkyle substitué ou non substitué ; et (c) un groupe alcényle substitué ou non substitué ;
$X^C$ représente un atome d'oxygène ou de soufre, et
dans laquelle $R^{3C}$ et $R^{4C}$ pris avec l'atome de carbone auquel ils sont liés peuvent former une structure cyclique à 5 à 10 chaînons, et dans laquelle $R^{3C}$ est un atome d'hydrogène, $R^{4C}$ est un groupe autre qu'un groupe méthyle,
pour la préparation d'une composition pharmaceutique destinée au traitement et/ou à la prévention des maladies des organes du système digestif.

**21.** Utilisation selon la revendication 20, dans laquelle la maladie des organes du système digestif est une indigestion, la constipation, la diarrhée, la cibophobie ou le syndrome de malabsorption.

FIG. 1

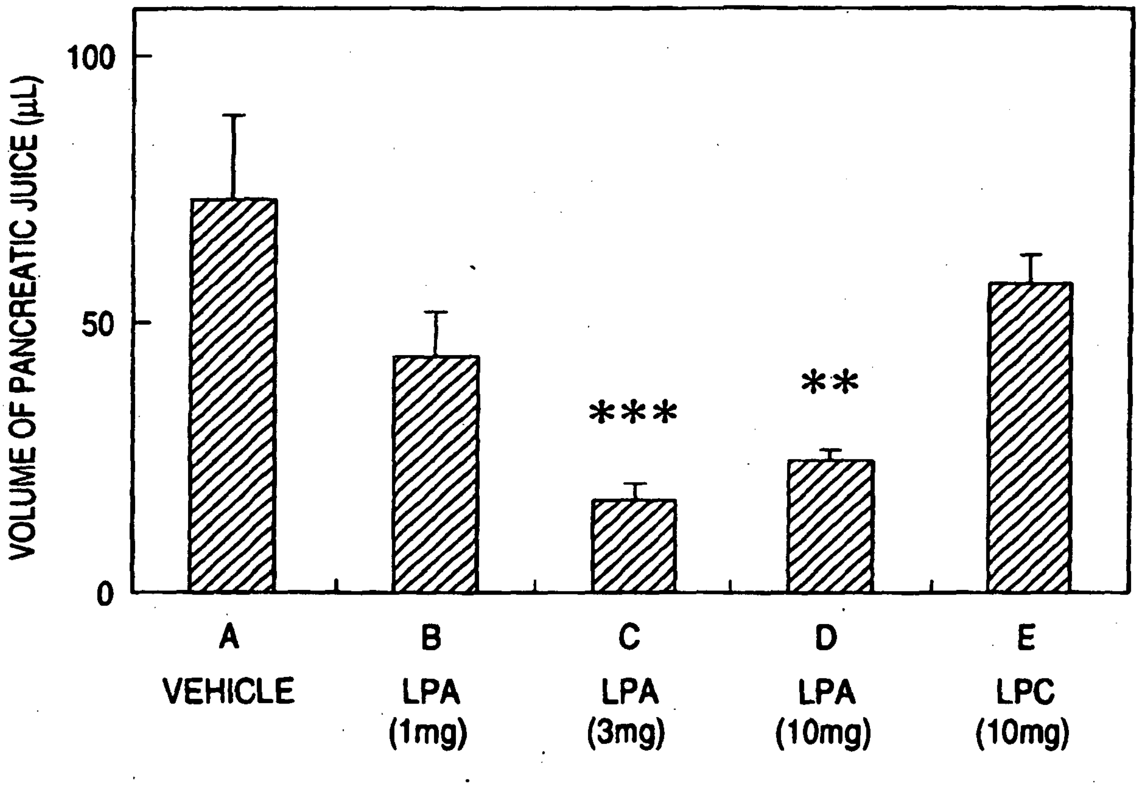


(MEAN ±S.E **: p<0.01, ***: p<0.001)

*FIG. 2*

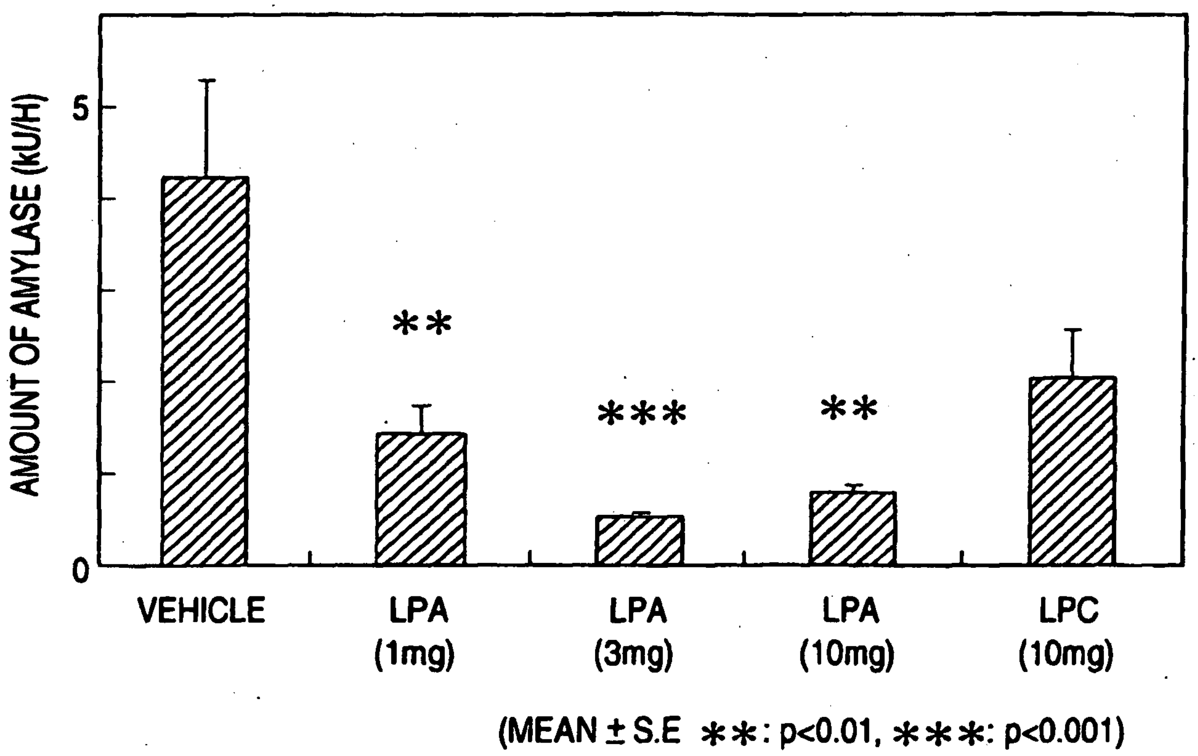
AMOUNT OF AMYLASE (kU/H)
5
0
**
***
**
VEHICLE
LPA (1mg)
LPA (3mg)
LPA (10mg)
LPC (10mg)
(MEAN ± S.E **: p<0.01, ***: p<0.001)

FIG. 3

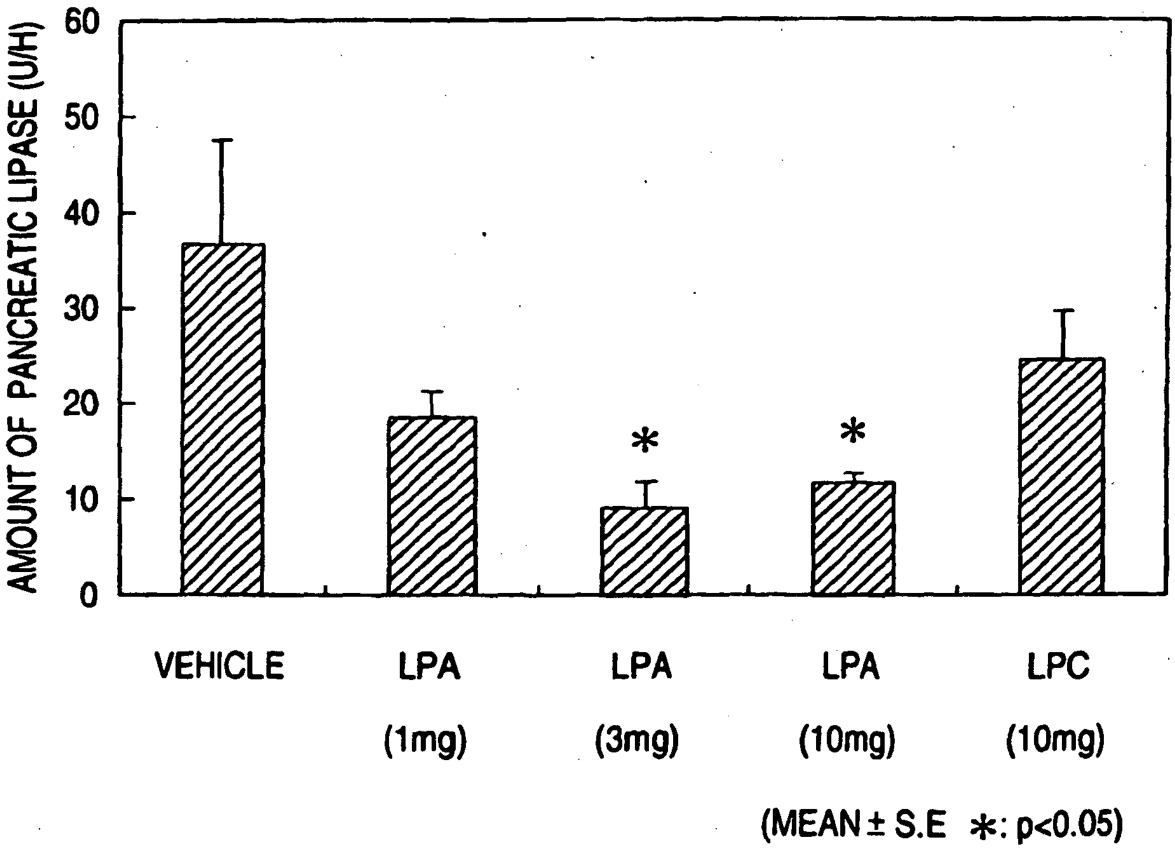
AMOUNT OF PANCREATIC LIPASE (U/H)
60
50
40
30
20
10
0
*
*
VEHICLE
LPA (1mg)
LPA (3mg)
LPA (10mg)
LPC (10mg)
(MEAN ± S.E *: p<0.05)

FIG. 4

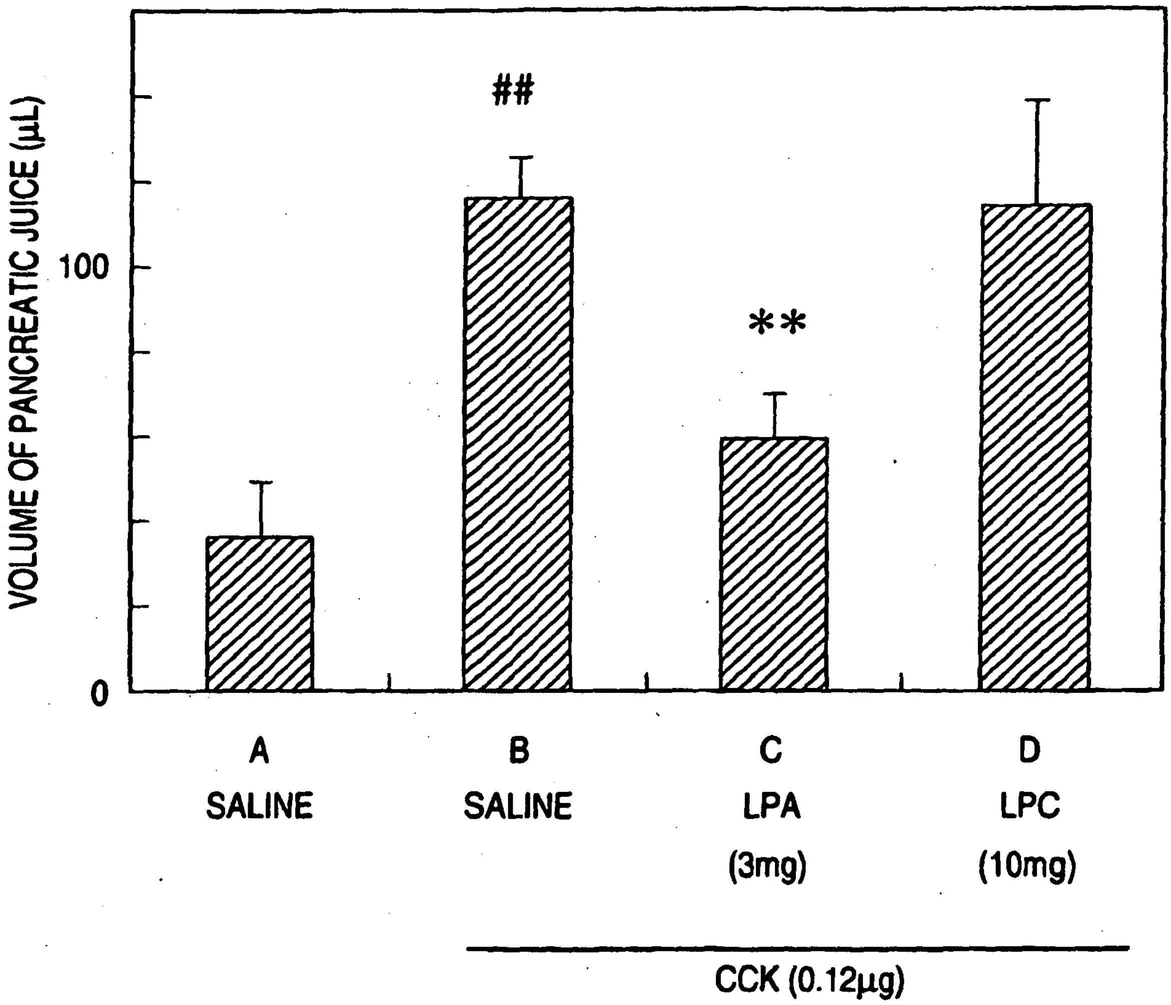


(MEAN±S.E **: p<0.01 vs. SALINE WITH CCK
##: p<0.01 vs. SALINE WITHOUT CCK)

FIG. 5

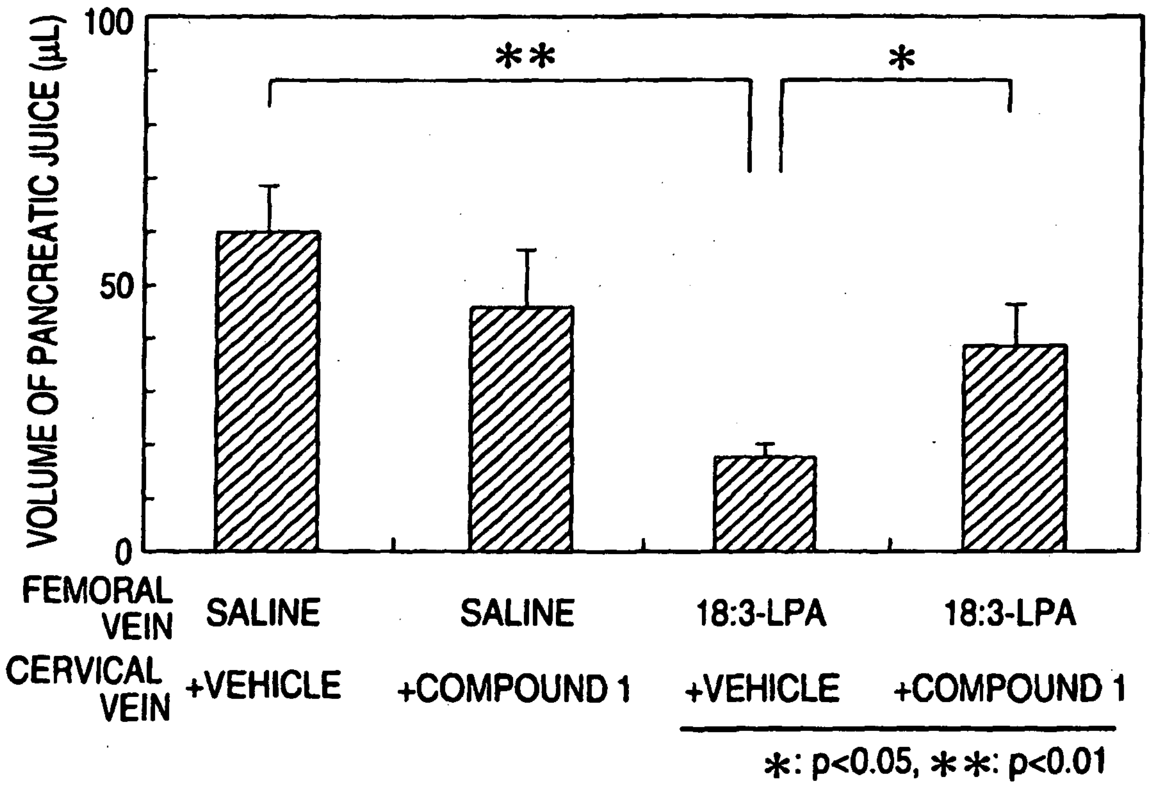
VOLUME OF PANCREATIC JUICE (µL)
100
50
0
**
*
FEMORAL VEIN
SALINE
SALINE
18:3-LPA
18:3-LPA
CERVICAL VEIN
+VEHICLE
+COMPOUND 1
+VEHICLE
+COMPOUND 1
*: p<0.05, **: p<0.01

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6380177 B
- WO 0160819 A
- US 2868691 A
- US 3095355 A


### Non-patent literature cited in the description


- *Mol Pharmacol,* December 2000, vol. 58 (6), 1188-96
- *J. Pharm. Pharmcol.,* 1991, vol. 43, 774
- *J. Pharm. Pharmacol.,* 1982, vol. 34, 514
- *BBRC,* 1996, vol. 218, 132
- *J. Cell Phys.,* 2001, vol. 186, 53
- *Biochem. and Biophys. Res. Commun.,* 1997, vol. 231, 619
- *J. Biol. Chem.,* 1999, vol. 274 (39), 2776
- Illustrative Digestive Organ Diseases, Series 14. Pancreatitis and Pancreatic Cancer. Medical View Co, vol. 90
- *Gendai Iryo,* 1984, vol. 16, 844
- *Kan Tan Sui,* 1998, vol. 37 (5), 703-728
- *Expert Opinion Pharmacother,* May 2000, vol. 1 (4), 841-847
- *Molecular Pharmacology,* 2001, vol. 60 (6), 1173-1180
- *Mol. Pharmacol.,* 2001, vol. 60 (6), 1173-1180